# EUROPEAN PATENT SPECIFICATION

(11) **EP 3 368 582 B1**
(45) Date of publication and mention of the grant of the patent: **30.12.2020**
(21) Application number: 16858507.3
(22) Date of filing: 28.10.2016
(51) Int. Cl.: C08G 18/10, C08G 18/12, C08G 18/32, C08G 18/48, A61L 27/18, A61F 2/24, C08G 81/02, C08G 77/458, C08L 83/12

(54) **POLYURETHANE/UREA MATERIALS**
POLYURETHAN-/HARNSTOFFMATERIALIEN
MATÉRIAUX DE POLYURÉTHANNE/URÉE

(30) Priority: 29.10.2015 AU 2015904428
(43) Date of publication of application: 05.09.2018
(60) Divisional application: 20209436.3
(73) Proprietor: Commonwealth Scientific and Industrial Research Organisation, Acton, ACT 2601 (AU)
(72) Inventor: GUNATILLAKE, Pathiraja Arachchillage, Mulgrave, Victoria 3170 (AU); BOWN, Mark, Notting Hill, Victoria 3168 (AU); ADHIKARI, Raju, Glen Waverley, Victoria 3150 (AU)
(74) Representative: Mewburn Ellis LLP
(86) International application number: PCT/AU2016/051019
(87) International publication number: WO 2017/070743

(56) References cited:
- EP-A1- 2 404 950
- EP-A1- 2 495 270
- WO-A1-2009/081681
- WO-A2-2006/002045
- JP-A- 2005 263 988
- US-A- 4 382 144
- US-A1- 2007 027 285
- US-A1- 2011 207 897
- US-B1- 6 313 254
- JANUSZ KOZAKIEWICZ ET AL: "Water-cured poly(urethane-urea)s containing soft segments originating from siloxane/carbonate macrodiols", POLIMERY, vol. 57, no. 11/12, 1 November 2012 (2012-11-01), pages 791-798, XP055578471, PL ISSN: 0032-2725, DOI: 10.14314/polimery.2012.791
- FANG, H.-X. et al.: "MICROPHASE SEPARATED STRUCTURES AND PROPERTIES OF PDMS-MDI-PEG COPOLYMER SURFACE", Chinese Journal of Polymer Science, vol. 27, no. 3, 2009, pages 327-334, XP055378920,
- SMETANKINA, N. P. et al.: "Carbofunctional organosilicon compounds. XII. Synthesis of siloxane-urethane oligomers with terminal isocyanate and hydroxyl groups", Sintez i fiziko-chimiya polimerov, vol. 8, 1971, pages 34-36, XP009510361,
- BARSZCZEWSKA-RYBAREK, I.: "Study of the effect of urethane-dimethacrylates structure on the morphology and properties of polymers based on them", Polimery, vol. 53, no. 3, 2008, pages 190-194, XP055378922,

## Description

### TECHNICAL FIELD

The present disclosure relates to soft block copolymer segments for a thermoplastic polyurethane or polyurethaneurea elastomer material and their reaction products with divalent compounds, such as diisocyanates, chain extenders and optional additional polyols or polyamines. Also disclosed are methods for the preparation of the soft block copolymer segments and reaction products, and the use of these components in the manufacture of materials such as biomaterials for articles, including medical devices such as implants.

### BACKGROUND

Previously, polyurethanes based on two or more macrodiols which are chemically different have been reported. This has been achieved by using a mixture or a blend of the macrodiols for making the polyurethane, and employing either a one-step or a two-step polymerisation procedure. This approach has a number of disadvantages including:
- where the macrodiols are not miscible, the resulting polyurethane is compositionally heterogeneous;
- only a limited number of macrodiol combinations can be used to make polyurethanes with good mechanical properties, and the segments from each of the macrodiols are randomly distributed within the polyurethane chain (often resulting in polyurethanes with poor mechanical properties); and
- the incorporation of higher molecular weight macrodiols or macrodiamines are limited to polyols such as polyethers, polyesters or polycarbonates.

EP2495270A describes a process for producing a polyurethane comprising using a polyester polyol, a polyether polyol, an isocyanate compound and a chain extender as a raw material to produce a polyurethane.

EP2404950A describes polyester-polysiloxane block copolymers which contain polysubstituted lactones as monomer units. A process for the preparation of the polyester-polysiloxane block copolymers using polysubstituted lactones is also described.

There is a need for identifying alternative block copolymers for use in polyurethane or polyurethaneurea elastomer materials in order to prepare various polymer products exhibiting a broad range of mechanical properties. The alternative block copolymers may address one or more disadvantages of previous approaches.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each claim of this application.

### SUMMARY

The present disclosure relates to macrodiols and macrodiamines which are chemically linked (linked-macrodiols or macrodiamines) to contain one or more chemically distinct moieties within a polymeric backbone. These macrodiols and macrodiamines may be useful in formulating polyurethanes/ureas for biomedical and non-biomedical applications.

In a first aspect, there is provided a copolymer segment which is a block copolymer macro-monomer of Formula 1 for a thermoplastic polyurethane or polyurethaneurea elastomer material: 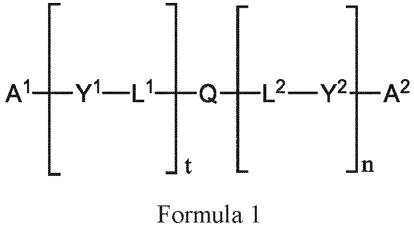 wherein
A¹ is an endcapping group;
A² is hydrogen or an endcapping group;
each Y¹ and Y² are independently selected from: a polysiloxane macrodiol, polysiloxane macrodiamine; polyether macrodiol, polycarbonate macrodiol, polyester macrodiol, and a polyhydrocarbon macrodiol;
each L¹ and L² is a divalent linking group independently selected from urethane, urea and phosphonate;
n is an integer of 1 to 5;
t is an integer of 0 to 5; and
Q is selected from a moiety of Formula A or Formula B: 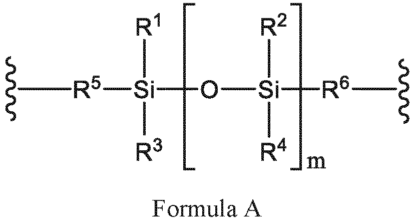 wherein
   R¹, R², R³, and R⁴, are each independently selected from hydrogen and an optionally substituted straight chain, branched or cyclic, saturated and unsaturated hydrocarbon radical;
   R⁵ and R⁶ are each independently selected from a straight chain, branched or cyclic, saturated and unsaturated hydrocarbon radical optionally interrupted with one or more heteroatoms independently selected from O, N and S;
   m is an integer of 2 to 50; or 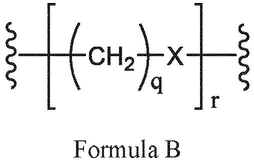 wherein
      X is a group selected from OC(O)O, C(O)O and O;
      q is an integer of 1 to 50; and
      r is an integer of 2 to 50,
wherein optionally substituted means optionally substituted with one or more substituents independently selected from: oxygen, nitrogen, sulphur, alkyl, alkenyl, alkynyl, aryl, halo, haloalkyl, haloalkenyl, haloalkynyl, haloaryl, hydroxy, alkoxy, alkenyloxy, alkynyloxy, aryloxy, carboxy, benzyloxy, haloalkoxy, haloalkenyloxy, haloalkynyloxy, haloaryloxy, nitro, nitroalkyl, nitroalkenyl, nitroalkynyl, nitroaryl, nitroheterocyclyl, azido, amino, alkylamino, alkenylamino, alkynylamino, arylamino, benzylamino, acyl, alkenylacyl, alkynylacyl, arylacyl, acylamino, acyloxy, aldehydo, alkylsulphonyl, arylsulphonyl, alkylsulphonylamino, arylsulphonylamino, alkylsulphonyloxy, arylsulphonyloxy, heterocyclyl, heterocycloxy, heterocyclylamino, haloheterocyclyl, alkylsulphenyl, arylsulphenyl, carboalkoxy, carboaryloxy, mercapto, alkylthio, arylthio, and acylthio groups.

In a second aspect, there is provided a thermoplastic polyurethane or polyurethaneurea elastomer material comprising a plurality of soft segments and hard segments, wherein the plurality of soft segments are each derived from at least one copolymer segment of Formula 1 according to the first aspect, or any embodiments thereof described herein, and optionally an additional polyol or polyamine, with the proviso that the polyurethane or polyurethaneurea elastomer material is not crosslinked.

In a third aspect, there is provided a thermoplastic polyurethane or polyurethaneurea elastomer material comprising a reaction product of:
i. at least one copolymer segment of Formula 1 according to the first aspect, or any embodiments thereof described herein;
ii. a diisocyanate;
iii. one or more chain extenders; and
iv. optionally an additional polyol or polyamine,
with the proviso that the polyurethane or polyurethaneurea elastomer material is not crosslinked.

In a fourth aspect, there is provided an article which is composed wholly or partly of the polyurethane or polyurethaneurea elastomeric material according to the second or third aspect, or any embodiments thereof described herein.

Also disclosed herein, there is provided a process for preparing the copolymer segment of Formula 1 according to the first aspect, or any embodiments thereof described herein, whereby the process comprises the step of combining:
i. at least one macrodiol; or
ii. at least one macrodiamine; or
iii. a mixture of at least one macrodiol and at least one macrodiamine,
with a divalent linking compound.

In a fifth aspect, there is provided a process for preparing a thermoplastic polyurethane or polyurethaneurea elastomer comprising the copolymer segment of Formula 1 according to the first aspect, or any embodiments thereof described herein, whereby the process comprises the steps of:
i. providing a copolymer segment of Formula 1 according to the first aspect, or any embodiments thereof described herein;
ii. optionally reacting the composition of step i. with a divalent compound and: at least one macrodiol; at least one macrodiamine; or a mixture of at least one macrodiol and at least one macrodiamine; and
iii. reacting the copolymer segment of step i. or step ii. with a chain extender or a mixture of chain extenders to form the polyurethane or polyurethaneurea elastomer.

### BRIEF DESCRIPTION OF THE DRAWINGS

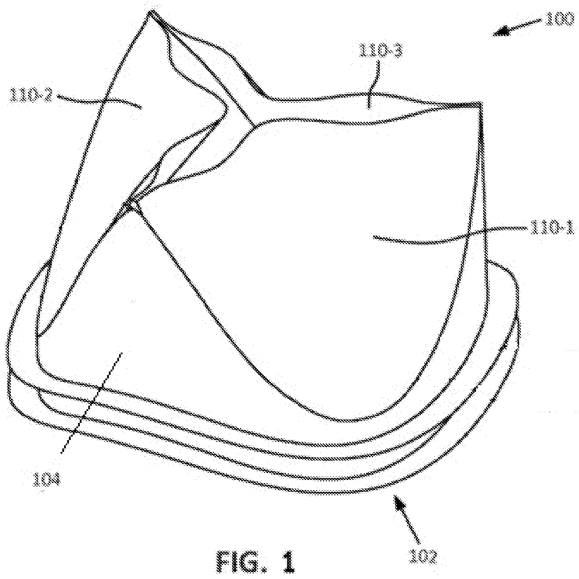

Fig. 1 is a perspective view of an example embodiment of a prosthetic heart valve incorporating polymers disclosed herein.

### DESCRIPTION OF EMBODIMENTS

The present disclosure describes the following various non-limiting embodiments, which relate to investigations undertaken to identify alternative blockcopolymer segments for use in polyurethane/urea materials and polymer products thereof.

It was surprisingly found that the copolymer segments disclosed herein can provide a number of advantages including enhanced soft segment properties in polyurethane/urea thermoplastic polymers. Enhanced soft segment properties include an improved miscibility with hard segments and increased inter molecular hydrogen bonding.

According to at least some embodiments described herein, the block copolymers, polyurethane/urea materials and polymer products thereof can provide advantages including:
- allowing a wide range of macrodiols, including those that are not miscible with each other, to be linked to produce a raft of different linked macrodiols which act as "macro-monomers";
- the formulation of polyurethanes with low modulus, high elasticity and high tear strength for use in cardio vascular medical implants;
- by utilising certain linker molecules, the synthesis of the linked-macrodiol can be prepared prior to the polyurethane/polyurethaneurea synthesis, without involving any purification steps; and
- the ability to increase the molecular weight of a "macro-monomer" by linking two or more macrodiols or diamine molecules, allows for the formulation of materials with a wide range of mechanical properties, via the variation in the relative proportions of 'soft' segments (for example a segment derived from the macrodiol or macrodiamine), and hard segments (for example a segment derived from the diisocyanate and an optional chain extender).

### TERMS

With regards to the definitions provided herein, unless stated otherwise, or implicit from context, the defined terms and phrases include the provided meanings. Unless explicitly stated otherwise, or apparent from context, the terms and phrases below do not exclude the meaning that the term or phrase has acquired by a person skilled in the relevant art. The definitions are provided to aid in describing particular embodiments, and are not intended to limit the claimed invention, because the scope of the invention is limited only by the claims. Furthermore, unless otherwise required by context, singular terms shall include pluralities and plural terms shall include the singular.

Herein, the term "polyurethane" relates to a polymer chain that comprises urethane (carbamate, -NH-COO-) links which connect monomer or "macro-monomer" units. Polyurethanes can be produced via the reaction of molecules containing a minimum of two isocyanate functional groups with other molecules which contain at least two alcohol (hydroxyl) groups.

Herein, the term "polyureas" relates to a polymer chain that comprises urea links (-NH-CO-NH-) which connect monomer or "macro-monomer" units. Polyureas can be produced via the reaction of molecules containing a minimum of two isocyanate groups with other molecules which contain at least two amine groups.

Herein, the term "polyurethaneurea" relates to a polymer chain that comprises both urethane and urea linking groups.

Herein, the term "macrodiol" refers to a polymeric material comprising two hydroxyl groups. For example, a copolymer segment of Formula 1 with two hydroxyl groups.

Herein, the term "macrodiamine" refers to a polymeric material comprising two amine groups. For example, a copolymer segment of Formula 1 with two amine groups.

Herein, the term "polyhydrocarbon macrodiol" refers to a polymeric material with a polymer backbone consisting entirely of hydrogen and carbon, and two hydroxyl groups. Examples include, but are not limited to: poly(isobutylene)diol, poly(butadiene)diol and hydrogenated poly(butadiene diol).

The term "macro-monomer" refers to a polymeric substance that possesses at least one polymerisable group, for example a hydroxyl group, which is capable of reacting with another compound, for example a diisocyanate.

Throughout the present specification, various aspects and components of the invention can be presented in a range format. The range format is included for convenience and should not be interpreted as an inflexible limitation on the scope of the invention. Accordingly, the description of a range should be considered to have specifically disclosed all the possible sub-ranges as well as individual numerical values within that range, unless specifically indicated. For example, description of a range such as from 1 to 5 should be considered to have specifically disclosed sub-ranges such as from 1 to 3, from 1 to 4, from 1 to 5, from 2 to 4, from 2 to 5, from 3 to 5 etc., as well as individual and partial numbers (except where integers are required), within the recited range, for example, 1, 2, 3, 4, 5, 5.5 and 6. This applies regardless of the breadth of the disclosed range. Where specific values are required, these will be indicated in the specification.

Herein, the term "hydrocarbon radical" refers to an optionally substituted straight chain, branched or cyclic, saturated and unsaturated hydrocarbon radicals. The hydrocarbon radicals (for example for substituents R¹, R², R³, R⁴, R⁵, R⁶, R⁷, R⁸, R⁹, R¹⁰, R¹¹ and/or R¹²) includes optionally substituted alkyl, alkenyl, alkynyl, aryl or heterocyclyl radicals.

The term "alkyl" denotes straight chain, branched or mono- or poly- cyclic alkyl, for example C₁₋₁₈alkyls or C₃₋₈cycloalkyls. "C₁₋₁₈ alkyls" and "C₃₋₈cycloalkyls" refer to alkyl groups having 1 to 18 carbon atoms or cycloalkyl groups having 3 to 8 carbon atoms.

As understood by a person skilled in the art, the term "C₁₋₁₈alkyls" means alkyl groups with 1, 2, 3, 4, 5, 6, 7, 8, 9, 10, 11, 12, 13, 14, 15, 16, 17 or 18 carbon atoms or a range comprising any of two of those integers and including: 1-2, 1-3, 1-4, 1-5, 1-6, 1-7, 1-8, 1-9, 1-10, 1-11, 1-12, 1-13, 1-14, 1-15, 1-16, 1-17, or 1-18 carbon atoms. Examples of straight chain and branched alkyl groups include optionally substituted: methyl, ethyl, propyl, isopropyl, butyl, isobutyl, sec-butyl, amyl, isoamyl, sec-amyl, 1,2-dimethylpropyl, 1,1-dimethylpropyl, pentyl, hexyl, 4-methylpentyl, 1-methylpentyl, 2-methylpentyl, 3-methylpentyl, 1,1-dimethylbutyl, 2,2-dimethylbutyl, 3,3-dimethylbutyl, 1,2-dimethylbutyl, 1,3-dinethylbutyl, 1,2,2-trimethylpropyl, or 1,1,2-trimethylpropyl, heptyl, 5-methylhexyl, 1-methylhexyl, 2,2-dimethylpentyl, 3,3-dimethylpentyl, 4,4-dimethylpentyl, 1,2-dimethylpentyl, 1,3-dimethylpentyl, 1 ,4-dimethylpentyl, 1,2,3-trimethylbutyl, 1,1,2-trimethylbutyl, 1,1,3-trimethylbutyl, octyl, 6-methylheptyl, 1-methyheptyl and 1,1,3,3-tetramethylbutyl groups, nonyl, 1-, 2-, 3-, 4-, 5-, 6- or 7-methyloctyl, 1-, 2-, 3-, 4- or 5-ethylheptyl, 1-, 2- or 3-propylhexyl, decyl, 1-, 2-, 3-, 4-, 5-, 6-, 7- or 8-methylnonyl, 1 -, 2-, 3-, 4-, 5- or 6-ethyloctyl, 1-, 2-, 3-, or 4-propylheptyl, undecyl, 1-, 2-, 3-, 4-, 5-, 6-, 7-, 8- or 9-methyldecyl, 1 -, 2-, 3-, 4-, 5-, 6- or 7-ethylnonyl. 1-, 2-, 3-, 4- or 5-propyloctyl, 1-, 2- or 3-butylheptyl, 1-pentylhexyl, dodecyl, 1-, 2-, 3-, 4-, 5-, 6-, 7-, 8-, 9- or 10-methylundecyl, 1-, 2-, 3-, 4-, 5-, 6-, 7- or 8-ethyldecyl, 1-, 2-, 3-, 4-, 5- or 6-propylnonyl, 1-, 2-, 3- or 4-butyloctyl, 1,2-pentylheptyl, tridecyl, tetradecyl, pentadecyl, hexadecyl, heptadecyl, or octadecyl groups.

As understood by a person skilled in the art, the term "C₃₋₈cycloalkyls" means cycloalkyl groups with 3, 4, 5, 6, 7 or 8 carbon atoms or a range comprising any of two of those integers and including 3-4, 3-5, 3-6, 3-7, 3-8, 4-5, 4-6, 4-7, 4-8, 5-6, 5-7, 5-8, 6-7, 6-8 or 7-8 carbon atoms. Examples of cyclic alkyl groups include optionally substituted: cyclopropyl, cyclobutyl, cyclopentyl, cyclohexyl, cycloheptyl or cyclooctyl groups.

The term "alkenyl" relates to groups formed from straight chain, branched or mono- or poly-cyclic alkenes including ethylenically mono- or poly-unsaturated alkyl or cycloalkyl groups as defined above, for example C₂₋₁₈ alkenyls or C₃₋₈ cycloalkenyls. "C₂₋₁₈ alkenyls" or "C₃₋₈ cycloalkenyls" refer to alkenyl or cycloalkenyl groups having 2 to 18 carbon atoms or 3 to 8 carbon atoms, respectively.

As understood by a person skilled in the art, the term "C₂₋₁₈alkenyls" means alkenyl groups with 2, 3, 4, 5, 6, 7, 8, 9, 10, 11, 12, 13, 14, 15, 16, 17 or 18 carbon atoms or a range comprising any of two of those integers and including: 2-3, 2-4, 2-5, 2-6, 2-7, 2-8, 2-9, 2-10, 2-11, 2-12, 2-13, 2-14, 2-15, 2-16, 2-17, or 2-18 carbon atoms. As understood by a person skilled in the art, the term "C₃₋₈ cycloalkenyls" means cycloalkenyl groups with 3, 4, 5, 6, 7 or 8 carbon atoms or a range comprising any of two of those integers and including 3-4, 3-5, 3-6, 3-7, 3-8, 4-5, 4-6, 4-7, 4-8, 5-6, 5-7, 5-8, 6-7, 6-8 or 7-8 carbon atoms. Examples of alkenyl and cycloalkenyl groups include optionally substituted: vinyl, allyl, 1-methylvinyl, butenyl, iso-butenyl, 3 -methyl-2-butenyl, 1-pentenyl, cyclopentenyl, 1-methyl-cyclopentenyl, 1-hexenyl, 3-hexenyl, cyclohexenyl, 1-heptenyl, 3-heptenyl, 1-octenyl, cyclooctenyl, 1,3-butadienyl, 1,4-pentadienyl, 1,3-cyclopentadienyl, 1,3-hexadienyl, 1,4-hexadienyl, 1,3-cyclohexadienyl, 1,4-cyclohexadienyl, 1,3-cycloheptadienyl, 1,3,5-cycloheptatrienyl and 1,3,5,7-cyclooctatetraenyl groups.

The term "alkynyl" denotes groups formed from straight chain, branched, or mono- or poly-cyclic alkynes, for example C₂₋₁₈alkynyls or C₃₋₈cycloalkynyls. Examples of alkynyl groups include optionally substituted: ethynyl, 1-propynyl, 1 and 2-butynyl, 2-methyl-2-propynyl, 2-pentynyl, 3-pentynyl, 4-pentynyl, 2-hexynyl, 3-hexynyl, 4-hexynyl, 5-hexynyl groups, 10-undecynyl, 4-ethyl-1-octyn-3-yl, 7-dodecynyl, 9-dodecynyl, 10-dodecynyl, 3-methyl-2-dodecyn-3-yl, 2-tridecynyl, 11-tridecynyl, 3-tetradecynyl, 7-hexadecynyl and 3-octadecynyl.

The term "aryl" denotes single, polynuclear, conjugated and fused residues of aromatic hydrocarbons. Examples of aryl groups include optionally substituted: phenyl, biphenyl, terphenyl, quaterphenyl, phenoxyphenyl, naphthyl, tetrahydronaphthyl, anthracenyl, dihydroanthracenyl, benzanthracenyl, dibenzanthracenyl and phenanthrenyl groups.

The term "heterocyclyl" denotes mono- or poly-cyclic heterocyclyl groups containing at least one heteroatom selected from nitrogen, sulphur and oxygen. Suitable heterocyclyl groups include optionally substituted: N-containing heterocyclic groups, such as, unsaturated 3 to 6 membered heteromonocyclic groups containing 1 to 4 nitrogen atoms, for example, pyrrolyl, pyrrolinyl, imidazolyl, pyrazolyl, pyridyl, pyrimidinyl, pyrazinyl, pyridazinyl, triazolyl or tetrazolyl; saturated 3 to 6-membered heteromonocyclic groups containing 1 to 4 nitrogen atoms, such as pyrrolidinyl, imidazolidinyl, piperidino or piperazinyl; unsaturated condensed heterocyclic groups containing 1 to 5 nitrogen atoms, such as, indolyl, isoindolyl, indolizinyl, benzimidazolyl, quinolyl, isoquinolyl, indazolyl, benzotriazolyl or tetrazolopyridazinyl; unsaturated 3 to 6-membered heteromonocyclic group containing an oxygen atom, such as, pyranyl or furyl; unsaturated 3 to 6-membered hetermonocyclic group containing 1 to 2 sulphur atoms, such as, thienyl; unsaturated 3 to 6-membered heteromonocyclic group containing 1 to 2 oxygen atoms and 1 to 3 nitrogen atoms, such as, oxazolyl, isoazolyl or oxadiazolyl; saturated 3 to 6-membered heteromonocyclic group containing 1 to 2 oxygen atoms and 1 to 3 nitrogen atoms, such as, morpholinyl; unsaturated condensed heterocyclic group containing 1 to 2 oxygen atoms and 1 to 3 nitrogen atoms, such as, benzoxazolyl or benzoxadiazolyl; unsaturated 3 to 6-membered heteromonocyclic group containing 1 to 2 sulphur atoms and 1 to 3 nitrogen atoms, such as thiazolyl or thiadiazolyl; saturated 3 to 6-membered heteromonocyclic group containing 1 to 2 sulphur atoms and 1 to 3 nitrogen atoms, such as, thiadiazolyl; and unsaturated condensed heterocyclic group containing 1 to 2 sulphur atoms and 1 to 3 nitrogen atoms, such as benzothiazolyl or benzothiadiazolyl groups.

Herein, "optionally substituted" means that a group may or may not be further substituted with one or more groups selected from: oxygen, nitrogen, sulphur, alkyl, alkenyl, alkynyl, aryl, halo, haloalkyl, haloalkenyl, haloalkynyl, haloaryl, hydroxy, alkoxy, alkenyloxy, alkynyloxy, aryloxy, carboxy, benzyloxy, haloalkoxy, haloalkenyloxy, haloalkynyloxy, haloaryloxy, nitro, nitroalkyl, nitroalkenyl, nitroalkynyl, nitroaryl, nitroheterocyclyl, azido, amino, alkylamino, alkenylamino, alkynylamino, arylamino, benzylamino, acyl, alkenylacyl, alkynylacyl, arylacyl, acylamino, acyloxy, aldehydo, alkylsulphonyl, arylsulphonyl, alkylsulphonylamino, arylsulphonylamino, alkylsulphonyloxy, arylsulphonyloxy, heterocyclyl, heterocycloxy, heterocyclylamino, haloheterocyclyl, alkylsulphenyl, arylsulphenyl, carboalkoxy, carboaryloxy, mercapto, alkylthio, arylthio, or acylthio groups. It will be appreciated that the term "optionally substituted" may also be referred to as "substituted or unsubstituted".

### BLOCK COPOLYMER SEGMENTS

In one aspect, there is provided copolymer segments of Formula 1 for a thermoplastic polyurethane or polyurethaneurea elastomer material: 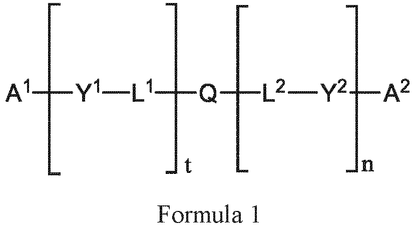 wherein
A¹ is an endcapping group;
A² is hydrogen or an endcapping group;
each Y¹ and Y² are independently selected from a polysiloxane macrodiol, polysiloxane macrodiamine; polyether macrodiol, polycarbonate macrodiol, polyester macrodiol, and a polyhydrocarbon macrodiol;
each L¹ and L² is a divalent linking group independently selected from urethane, urea and phosphonate;
n is an integer of 1 to 5;
t is an integer of 0 to 5; and
Q is selected from a moiety of Formula A or Formula B: 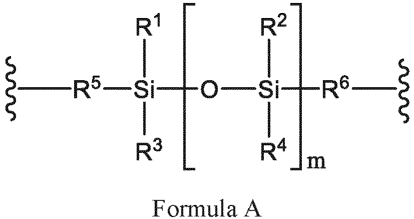 wherein
   R¹, R², R³, and R⁴, are each independently selected from hydrogen and an optionally substituted straight chain, branched or cyclic, saturated and unsaturated hydrocarbon radical;
   R⁵ and R⁶ are each independently selected from a straight chain, branched or cyclic, saturated and unsaturated hydrocarbon radical optionally interrupted with one or more heteroatoms independently selected from O, N and S;
   m is an integer of 2 to 50; or 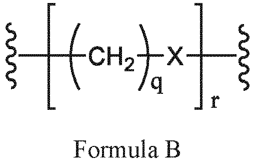 wherein
      X is a group selected from OC(O)O, C(O)O and O;
      q is an integer of 1 to 50; and
      r is an integer of 2 to 50,
wherein optionally substituted means optionally substituted with one or more substituents independently selected from: oxygen, nitrogen, sulphur, alkyl, alkenyl, alkynyl, aryl, halo, haloalkyl, haloalkenyl, haloalkynyl, haloaryl, hydroxy, alkoxy, alkenyloxy, alkynyloxy, aryloxy, carboxy, benzyloxy, haloalkoxy, haloalkenyloxy, haloalkynyloxy, haloaryloxy, nitro, nitroalkyl, nitroalkenyl, nitroalkynyl, nitroaryl, nitroheterocyclyl, azido, amino, alkylamino, alkenylamino, alkynylamino, arylamino, benzylamino, acyl, alkenylacyl, alkynylacyl, arylacyl, acylamino, acyloxy, aldehydo, alkylsulphonyl, arylsulphonyl, alkylsulphonylamino, arylsulphonylamino, alkylsulphonyloxy, arylsulphonyloxy, heterocyclyl, heterocycloxy, heterocyclylamino, haloheterocyclyl, alkylsulphenyl, arylsulphenyl, carboalkoxy, carboaryloxy, mercapto, alkylthio, arylthio, and acylthio groups.

The copolymer segment may be a block copolymer segment of Formula 1A: 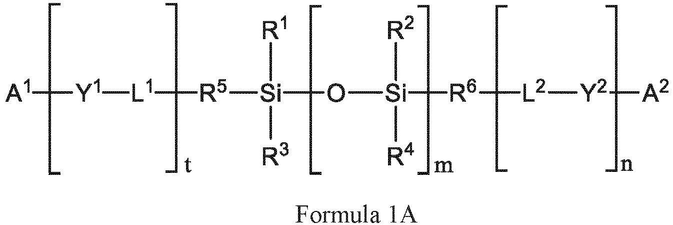 wherein each A¹, A², Y¹, L¹, L², Y², R¹, R², R³, R⁴, R⁵, R⁶, m, n and t, are as defined herein.

The copolymer segment may be a block copolymer segment of Formula 1A(i): 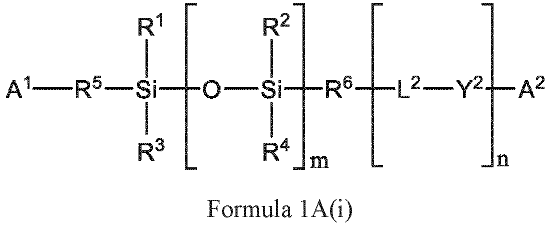 wherein each A¹, A², L², Y², R¹, R², R³, R⁴, R⁵, R⁶, m and n, are as defined herein.

The copolymer segment may be a block copolymer segment of Formula 1A(i)(a): 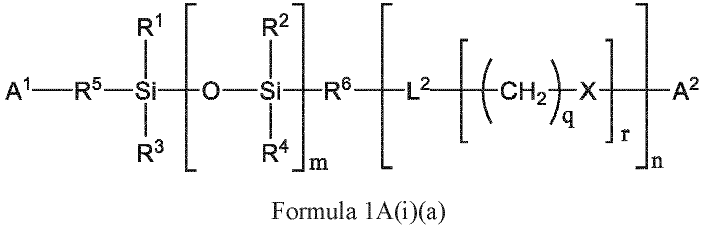 wherein each A¹, A², L², R¹, R², R³, R⁴, R⁵, R⁶, X, m, q and r, are as defined herein.

The copolymer segment may be a block copolymer segment of Formula 1A(i)(b): 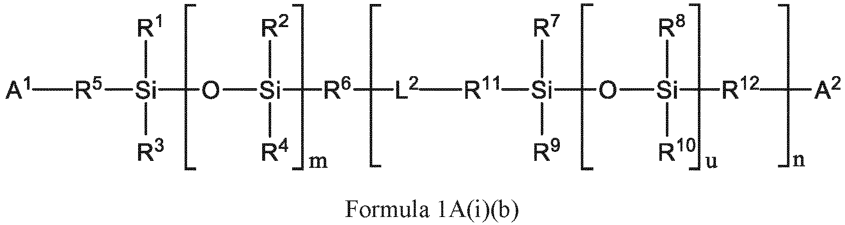 wherein each A¹, A², L², R¹, R², R³ , R⁴, R⁵ , R⁶, R⁷, R⁸ , R⁹ , R¹⁰, R¹¹, R¹², m, n and u, are as defined herein.

The copolymer segment may be a block copolymer segment of Formula 1A(ii): 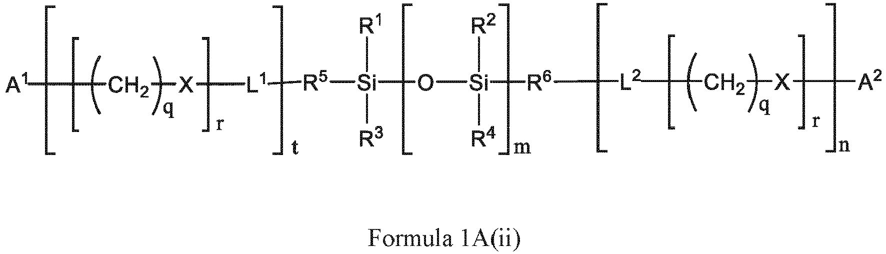 wherein each A¹, A², L¹, L², R¹, R², R³, R⁴, R⁵, R⁶, X, m, q, r and t, are as defined herein.

The copolymer segment may be a block copolymer segment of Formula 1B: 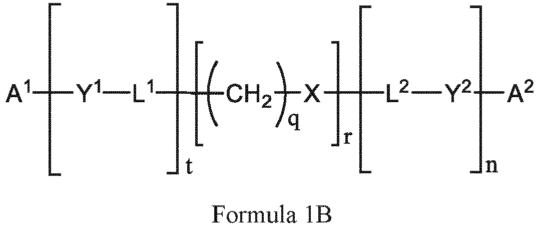 wherein each A¹, A², Y¹, L¹, L², Y², X, n, q, r and t are as defined herein.

The copolymer segment may be a block copolymer segment of Formula 1B(i): 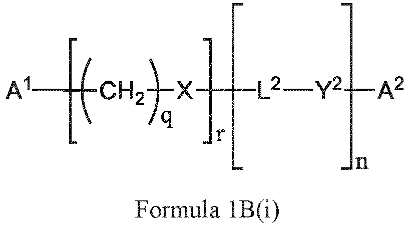 wherein each A¹, A², L², Y², X, n, q and r are as defined herein.

The copolymer segment may be a block copolymer segment of Formula 1B(i)(a): 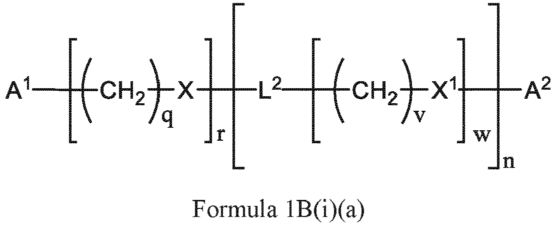 wherein
n is an integer from 1 to 5;
A¹ is an endcapping group;
A² is hydrogen or an endcapping group;
X and X¹ are each independently selected from OC(O)O, C(O)O and O;
q and v are each independently selected from an integer of 1 to 50 (for example 5 to 20); and
r and w are each independently selected from an integer of 2 to 50.

The copolymer segment may be a block copolymer segment of Formula 1B(i)(b): 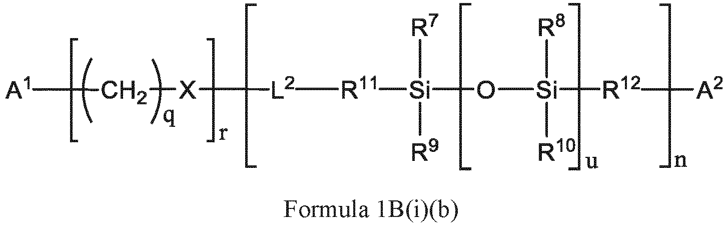 wherein each A¹, A², q, X, and r, are as defined in claim 1, and L², n, R⁷, R⁸, R⁹, R¹⁰, R¹¹, R¹² and u, are as defined herein.

The copolymer segment may be a block copolymer segment of Formula 1B(ii): 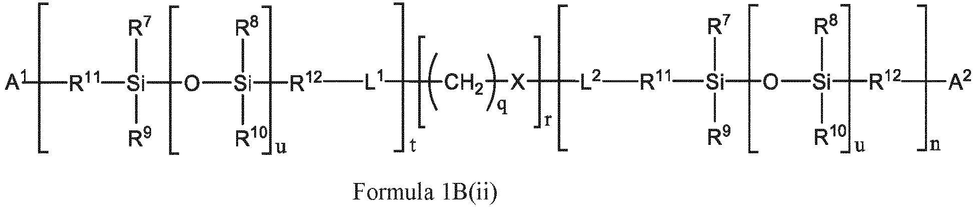 wherein each A¹, A², L¹, L², X, n, q, r and t, are as defined in claim 1, and each R⁷, R⁸, R⁹, R¹⁰, R¹¹, R¹² and u, are as defined herein.

### Substituent 0

Substituent Q may be selected from a moiety of Formula A or Formula B as described herein.

Substituent Q may be selected from a moiety of Formula A: 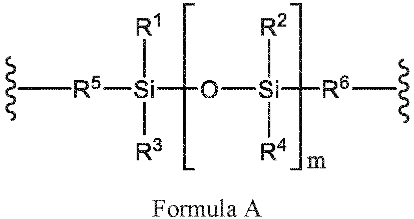 wherein
R¹, R², R³, and R⁴, are each independently selected from hydrogen and an optionally substituted straight chain, branched or cyclic, saturated and unsaturated hydrocarbon radical;
R⁵ and R⁶ are each independently selected from a straight chain, branched or cyclic, saturated and unsaturated hydrocarbon radical optionally interrupted with one or more heteroatoms independently selected from O, N and S;
m is an integer of 2 to 50.

Substituent Q may be selected from a moiety of Formula B: 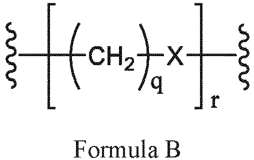 wherein
X is a group selected from OC(O)O, C(O)O and O;
q is an integer of 1 to 50; and
r is an integer of 2 to 50.

In one embodiment substituent Q is a moiety of Formula A. Q may be α,ω bis-(6-hydroxyethoxypropyl)polydimethylsiloxane. In another embodiment substituent Q is a moiety of Formula B.

### Substituent A¹

Herein, substituent A¹ is an "endcapping group" and includes reactive functional groups or groups containing reactive functional groups. Examples of suitable reactive functional groups for substituent A¹ include: hydroxyl groups, carboxylic acids, aldehydes, ketones, esters, acid halides, acid anhydrides, amine groups, imine groups, thio groups, thioesters, sulphonic acids and epoxides.

A¹ may be selected from hydroxyl or amine. In one embodiment, A¹ is a hydroxyl group. In another embodiment, A¹ is an amine group.

### Substituent A²

Herein substituent A² is hydrogen or an "endcapping group" and includes reactive functional groups or groups containing reactive functional groups. Examples of suitable reactive functional groups for substituent A² include: hydroxyl groups, carboxylic acids, aldehydes, ketones, esters, acid halides, acid anhydrides, amine groups, imine groups, thio groups, thioesters, sulphonic acids and epoxides.

A² may be selected from hydrogen, hydroxyl or amine. In one embodiment A² is hydrogen. In one embodiment A² is an endcapping group. In another embodiment A² is a hydroxyl group. In another embodiment A² is an amine group.

### Substituents Y¹ and Y²

Herein each individual substituent Y¹ and Y² may be independently selected from a polysiloxane macrodiol, polysiloxane macrodiamine, polyether macrodiol, polycarbonate macrodiol, polyester macrodiol, or a polyhydrocarbon macrodiol.

At least one of Y¹ and Y² may be a polysiloxane macrodiol. Y¹ may be a polysiloxane macrodiol. Y² may be a polysiloxane macrodiol.

Examples of polysiloxane macrodiols include, but are not limited to: polydimethylsiloxane diols, such as α,ω bis-(6-hydroxyethoxypropyl)polydimethylsiloxane, α,ω bis-(4-hydroxybutyl)polydimethylsiloxane and α,ω bis-(3-hydroxypropyl)polydimethylsiloxane.

In one embodiment at least one of Y¹ and Y² is a polydimethylsiloxane diol. In another embodiment both of Y¹ and Y² are polydimethylsiloxane diols. In another embodiment at least one of Y¹ and Y² is α,ω bis-(6-hydroxyethoxypropyl)polydimethylsiloxane. In another embodiment both of Y¹ and Y² are α,ω bis-(6-hydroxyethoxypropyl)polydimethylsiloxane.

At least one of Y¹ and Y² may be a polysiloxane macrodiamine. Y¹ may be a polysiloxane macrodiamine. Y² may be a polysiloxane macrodiamine.

Examples of polysiloxane macrodiamines include, but are not limited to: α,ω bis-(aminomethyl)polydimethylsiloxane, α,ω bis-(2-aminoethyl)polydimethylsiloxane, α,ω bis-(3-aminopropyl)polydimethylsiloxane, α,ω bis-(4-aminobutyl)polydimethylsiloxane, α,ω bis-(5-aminopentyl)polydimethylsiloxane and the like.

At least one of Y¹ and Y² may be a polyether macrodiol. Y¹ may be a polyether macrodiol. Y² may be a polyether macrodiol.

Examples of polyether macrodiols include, but are not limited to: poly(ethylene oxide), poly(propylene oxide), poly(butylene oxide), poly(pentylene oxide), poly(hexamethylene oxide) (PHMO), poly(heptamethylene oxide), poly(octamethylene oxide) (POMO) and poly(decamethylene oxide) (PDMO).

In one embodiment at least one of Y¹ and Y² is poly(hexamethylene oxide) (PHMO).

In one embodiment both of Y¹ and Y² are poly(hexamethylene oxide) (PHMO).

At least one of Y¹ and Y² may be a polycarbonate macrodiol. Y¹ may be a polycarbonate macrodiol. Y² may be a polycarbonate macrodiol.

Examples of polycarbonate macrodiols include, but are not limited to: poly(propylene carbonate), poly(hexamethylene carbonate) and polycarbonate and copolycarbonate macrodiols can be prepared by using an ester interchange reaction as described in P. A. Gunatillake et al., Journal of Applied Polymer Science, 69(8) 1621-1633, 1998, for example by reacting a carbonate such as ethylene carbonate with a diol. Appropriate diols include, but are not limited to: 1,6-hexanediol, 1,10-decanediol, 2,2-diethyl-1,3-propanediol, 1,4-cyclohexanedimethanol and bis(4-hydroxybutyl)-1,1,3,3-tetramethyldisiloxane.

At least one of Y¹ and Y² may be a polyester macrodiol. Y¹ may be a polyester macrodiol. Y² may be a polyester macrodiol.

Examples of polyester macrodiols include, but are not limited to: poly(caprolactone) diol, poly(tetramethyleneadipate) diol, poly(D,L-lactide) and, poly(glycolide).

At least one of Y¹ and Y² may be a polyhydrocarbon macrodiol. Y¹ may be a polyhydrocarbon macrodiol. Y² may be a polyhydrocarbon macrodiol.

Examples of polyhydrocarbon macrodiols include polymeric aliphatic α,ω-diols. Specific examples of polyhydrocarbon macrodiols include, but are not limited to: poly(isobutylene)diol and poly(butadiene)diols, such as hydrogenated poly(butadiene)diols (including fully hydrogenated poly(butadiene)diols).

In one embodiment, Y¹ and Y² are each independently selected from a moiety of Formula A' or Formula B': 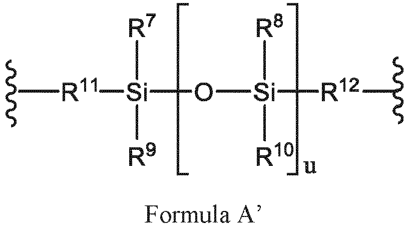 wherein
R⁷, R⁸, R⁹, and R¹⁰, are each independently selected from hydrogen and an optionally substituted straight chain, branched or cyclic, saturated and unsaturated hydrocarbon radical; and
R¹¹ and R¹² are each independently selected from a straight chain, branched or cyclic, saturated and unsaturated hydrocarbon radical optionally interrupted with one or more heteroatoms independently selected from O, N and S; and
u is an integer of 1 to 50; or 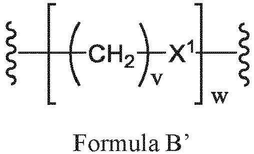 wherein
   X¹ is a group selected from OC(O)O, C(O)O and O;
   v is an integer of 1 to 50, for example an integer of 5 to 20; and
   w is an integer of 2 to 50.

In one embodiment at least one of Y¹ and Y² is a moiety of Formula A'.

In one embodiment at least one of Y¹ and Y² is a moiety of Formula B'.

In another embodiment substituents Y¹ and Y² are the same.

In yet another embodiment substituents Y¹ and Y² are different. It will be appreciated that Y¹ and Y² may be selected from different groups within same individual Formula A'. Similarly, it will also be appreciated that Y¹ and Y² may be selected from different groups within same individual Formula B'.

### Substituents L¹ and L²

Herein substituents L¹ and L² are divalent linking groups independently selected from urethane, urea and phosphonate linking groups.

At least one of L¹ or L² may be a urethane linking group. L¹ may be a urethane linking group. L² may be a urethane linking group.

Urethane linking groups can be produced by reacting hydroxyl containing compounds, such as a macrodiol, with a diisocyanate. Examples of appropriate diisocyanates include aliphatic, cyclic or aromatic diisocyanates such as, for example: 1,4-diisocyanatobutane, 1,12-diisocyanatododecane, 1,6-diisocyantehexane, 1,8-diisocyanateoctane, 4,4'-methylenediphenyl diisocyanate (methylenebis(cyclohexyl diisocyanate) (H12MDI), p-phenylene diisocyanate (p-PDI), m-phenylene diisocyanate (m-PDI) trans-cyclohexane-1,4-diisocyanate (CHDI) or a mixture of the *cis* and *trans* isomers, 1,6-hexamethylene diisocyanate (HDI), 2,4-toluene diisocyanate (2,4-TDI) or its isomers (for example 2,6-toluene diisocyanate (2,6-TDI)), or mixtures thereof, p-tetramethylxylene diisocyanate (p-TMXDI), isophorone diisocyanate or m-tetramethylxylene diisocyanate (m-TMXDI), 1,6-diisocyanatohexane (DICH), 1,3-bis(1-isocyanato-1-methylethyl)benzene, or 1,5-diisocyanatonaphthalene (NDI).

In one embodiment, L¹ or L² may be independently a linking group of Formula E: 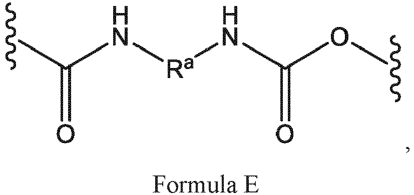 wherein R^{a} is selected from:
an optionally substituted straight chain, branched or cyclic, saturated or unsaturated hydrocarbon radical (including optionally substituted alkyl, alkenyl, alkynyl, aryl or heterocyclyl radicals).

For example, L¹ or L² may be selected from:

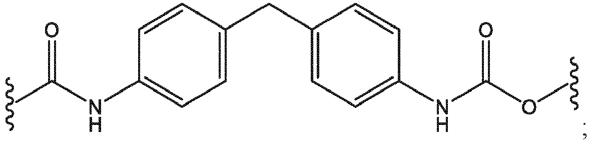

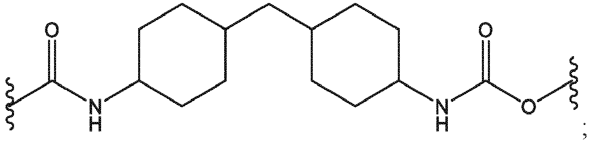

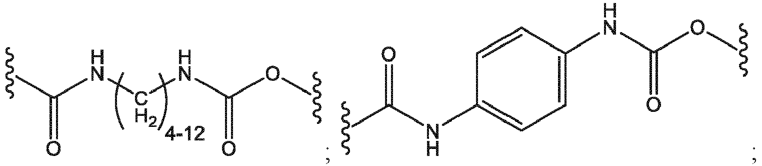

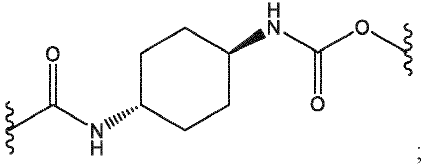

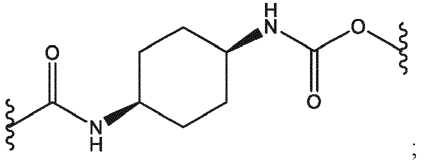

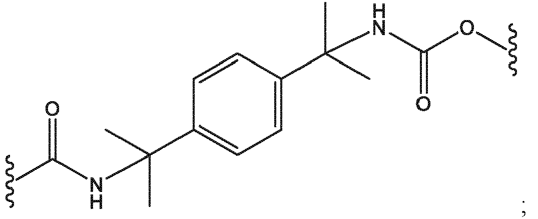

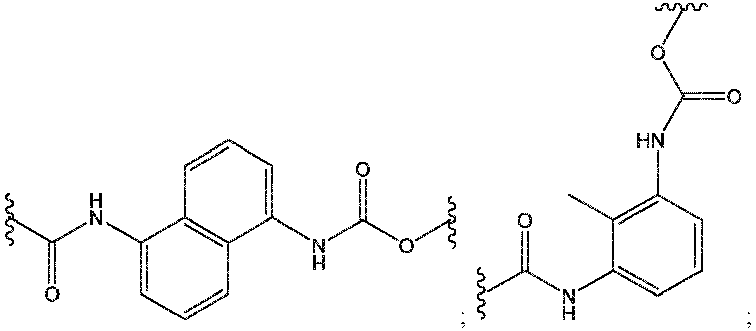

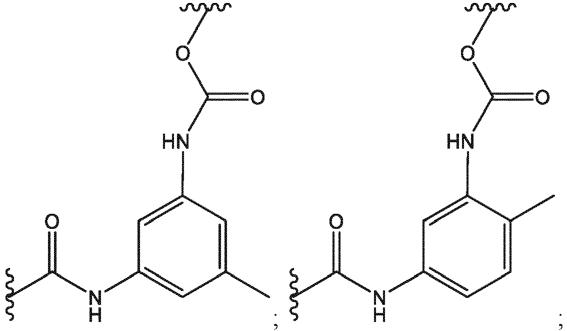

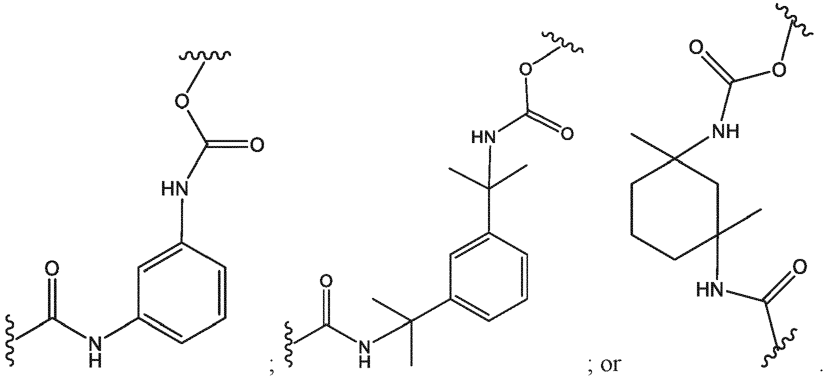

At least one of L¹ or L² may be a urea linking group. L¹ may be a urea linking group. L² may be a urea linking group.

Urea linking groups can be produced by reacting amine containing compounds, such as a macrodiamine, with a diisocyanate. Examples of appropriate diisocyanates include aliphatic, cyclic or aromatic diisocyanates such as, for example: 1,4-diisocyanatobutane, 1,12-diisocyanatododecane, 1,6-diisocyantehexane, 1,8-diisocyanateoctane, 4,4'-methylenediphenyl diisocyanate (MDI), 4,4'-methylenebis(cyclohexyl diisocyanate) (H12MDI), p-phenylene diisocyanate (p-PDI), m-phenylene diisocyanate (m-PDI) trans-cyclohexane-1,4-diisocyanate (CHDI) or a mixture of the *cis* and *trans* isomers, 1,6-hexamethylene diisocyanate (HDI), 2,4-toluene diisocyanate (2,4-TDI) or its isomers (for example 2,6-toluene diisocyanate (2,6-TDI)), or mixtures thereof, p-tetramethylxylene diisocyanate (p-TMXDI), isophorone diisocyanate or m-tetramethylxylene diisocyanate (m-TMXDI), or 1,5-diisocyanatonaphthalene (NDI).

In one embodiment, L¹ or L² may be independently a linking group of Formula F: 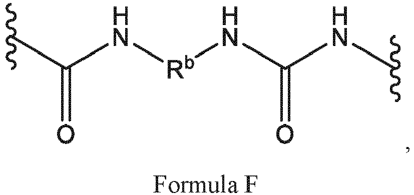 wherein R^{b} is selected from:
an optionally substituted straight chain, branched or cyclic, saturated or unsaturated hydrocarbon radical (including optionally substituted alkyl, alkenyl, alkynyl, aryl or heterocyclyl radicals).

For example, L¹ or L² may be selected from:

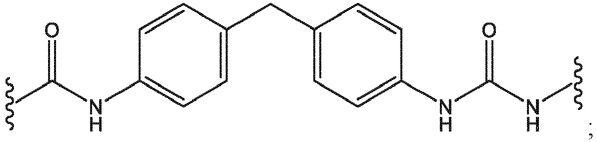

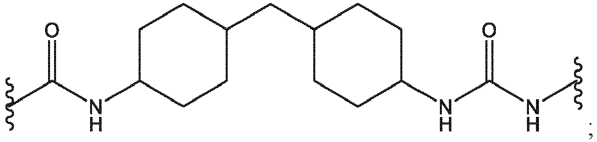

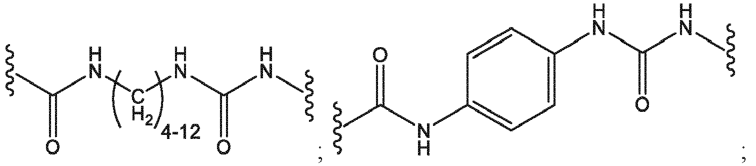

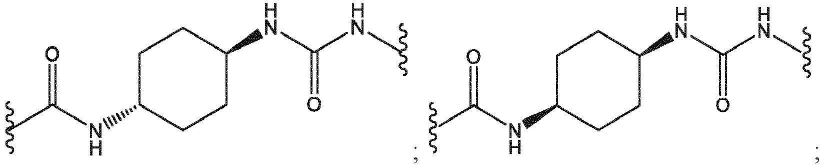

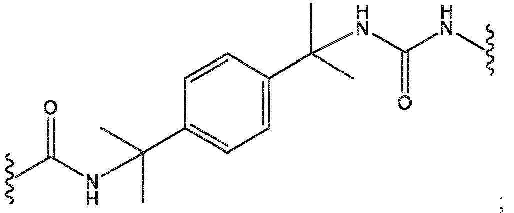

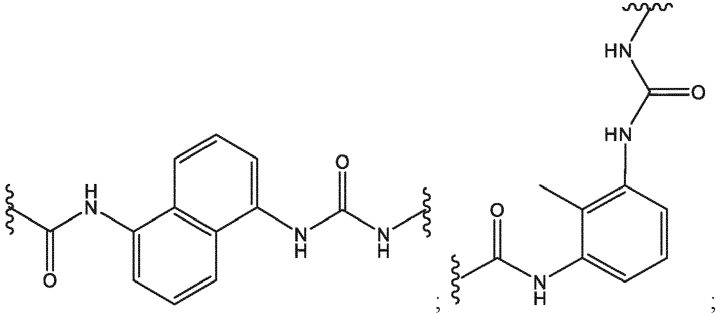

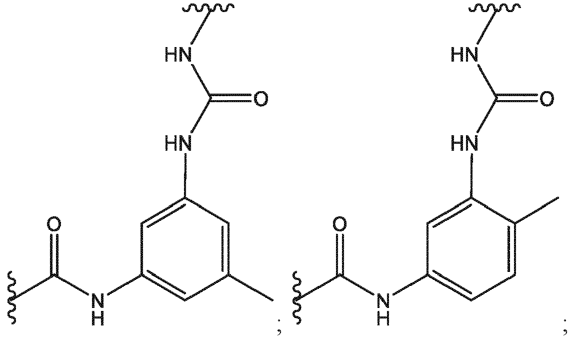

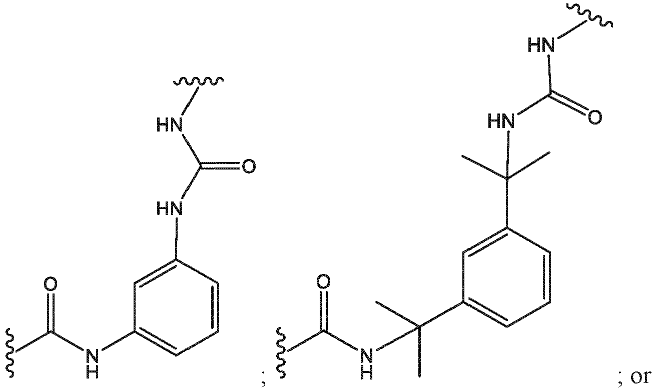

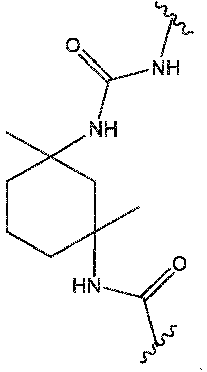

At least one of L¹ or L² may be a phosphonate linking group. L¹ may be a phosphonate linking group. L² may be a phosphonate linking group.

L¹ or L² may be independently a linking group of Formula H: 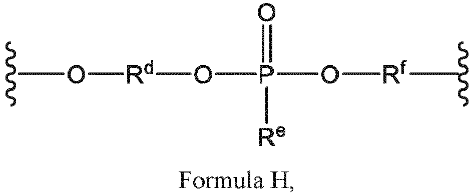 wherein:
each of R^{d}, and R^{f} is each independently selected from: an optionally substituted straight chain, branched or cyclic, saturated or unsaturated hydrocarbon radical (including optionally substituted alkyl, alkenyl, alkynyl, aryl or heterocyclyl radicals); and
R^{e} is selected from: hydrogen; or an optionally substituted straight chain, branched or cyclic, saturated or unsaturated hydrocarbon radical (including optionally substituted alkyl, alkenyl, alkynyl, aryl or heterocyclyl radicals). For example, substituent R^{e} may be an optionally substituted methyl group.

In one embodiment substituents L¹ and L² are the same. In another embodiment substituents L¹ and L² are different.

### Substituents R¹, R², R³, R⁴, R⁵ R⁶, R⁷, R⁸, R⁹, R¹⁰, R¹¹ and R¹²

Herein, R¹, R², R³, R⁴, R⁷, R⁸, R⁹, and R¹⁰ are each independently selected from hydrogen and an optionally substituted straight chain, branched or cyclic, saturated and unsaturated hydrocarbon radical (including optionally substituted alkyl, alkenyl, alkynyl, aryl or heterocyclyl radicals).

Herein, R⁵, R⁶, R¹¹ and R¹² are each independently selected from an optionally substituted straight chain, branched or cyclic, saturated and unsaturated hydrocarbon radical optionally interrupted with one or more heteroatoms independently selected from O, N and S (including optionally substituted alkyl, alkenyl, alkynyl, aryl or heterocyclyl radicals).

In one embodiment at least one of R¹, R², R³, and R⁴ is an optionally substituted alkyl group, including optionally substituted methyl, ethyl, propyl or butyl groups. For example at least one of R¹, R², R³, and R⁴ is a methyl group.

In one embodiment each of R¹, R², R³, and R⁴ is an optionally substituted alkyl group, including optionally substituted methyl, ethyl, propyl or butyl groups. For example each of R¹, R², R³, and R⁴ is a methyl group.

In one embodiment at least two of R¹, R², R³, and R⁴ are the same substituent.

In one embodiment all of R¹, R², R³, and R⁴ are the same substituent.

In another embodiment at least one of R⁵ and R⁶ is an optionally substituted alkyl or alkoxyalkyl group. For example at least one of R⁵ and R⁶ may be independently selected from optionally substituted: propylene, butylene, pentylene, hexylene, ethoxypropyl, propoxypropyl and butoxypropyl groups.

In another embodiment both of R⁵ and R⁶ are optionally substituted alkyl or alkoxyalkyl groups. For example both of R⁵ and R⁶ may be selected from optionally substituted: propylene, butylene, pentylene, hexylene, ethoxypropyl, propoxypropyl and butoxypropyl groups. For example both R⁵ and R⁶ may be ethoxypropyl.

In another embodiment at least one of R⁵ and R⁶ is independently selected from the moiety of Formula D': 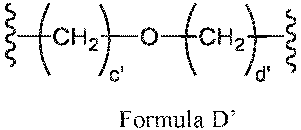 wherein c' is an integer between 1 and 6; and d' is an integer between 1 and 6. For example c' is 2 and/or d' is 3.

In one embodiment R⁵ and R⁶ are the same substituent.

In one embodiment R⁵ and R⁶ are different substituents.

In one embodiment at least one of R⁷, R⁸, R⁹, and R¹⁰ is an optionally substituted alkyl group, including optionally substituted methyl, ethyl, propyl or butyl groups. For example at least one of R⁷, R⁸, R⁹, and R¹⁰ is a methyl group.

In one embodiment each of R⁷, R⁸, R⁹, and R¹⁰ is an optionally substituted alkyl group, including optionally substituted methyl, ethyl, propyl or butyl groups. For example each of R⁷, R⁸, R⁹, and R¹⁰ is a methyl group.

In one embodiment at least two of R⁷, R⁸, R⁹, and R¹⁰ are the same substituent.

In one embodiment all of R⁷, R⁸, R⁹, and R¹⁰ are the same substituent.

In another embodiment at least one of R¹¹ and R¹² is an optionally substituted alkyl or alkoxyalkyl group. For example at least one of R¹¹ and R¹² may be independently selected from optionally substituted: propylene, butylene, pentylene, hexylene, ethoxypropyl, propoxypropyl and butoxypropyl groups.

In another embodiment both of R¹¹ and R¹² are optionally substituted alkyl or alkoxyalkyl groups. For example both of R¹¹ and R¹² may be selected from optionally substituted: propylene, butylene, pentylene, hexylene, ethoxypropyl, propoxypropyl and butoxypropyl groups. For example both R¹¹ and R¹² may be ethoxypropyl.

In one embodiment, at least one of R¹¹ and R¹² is independently selected from the moiety of Formula C': 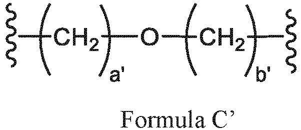 wherein a' is an integer between 1 and 6; and b' is an integer between 1 and 6. For example a' is 2 and/or b' is 3.

In one embodiment R¹¹ and R¹² are the same substituent. In another embodiment R¹¹ and R¹² are different substituents.

### Substituents X and X¹

Herein, substituent X and X¹ may be independently selected from OC(O)O, C(O)O and O.

X may be OC(O)O. X may be C(O)O. X may be O. X¹ may be OC(O)O. X¹ may be C(O)O. X¹ may be O. X and X¹ may be the same. X and X¹ may be different.

### Integers a', b', c', d', m, m', m", n, q, q', q", r, r', r", t, u, v and w

Integer a' may be: 1 or 2 or 3 or 4 or 5 or 6. In one embodiment, integer a' is 2. In another embodiment, integer a' is 3.

Integer b' may be: 1 or 2 or 3 or 4 or 5 or 6. In one embodiment, integer b' is 2. In another embodiment integer b' is 3.

Integer c' may be: 1 or 2 or 3 or 4 or 5 or 6. In one embodiment, integer c' is 2. In another embodiment, integer c' is 3.

Integer d' may be: 1 or 2 or 3 or 4 or 5 or 6. In one embodiment, integer d' is 2. In another embodiment integer d' is 3.

Herein integer m may be: 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

Herein integer m' may be: 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

Herein integer m" may be: 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

Herein integer n may be: 1 or 2 or 3 or 4 or 5.

Herein integer t may be: 0 or 1 or 2 or 3 or 4 or 5.

In one embodiment integer t is 0.

Herein integer q may be: 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

In one embodiment integer q may be 5 or 6 or 7 or 8 or 9 or 10 or 11 or 12 or 13 or 14 or 15 or 16 or 17 or 18 or 19 or 20.

Herein, q may be in a range of 1 to 10, for example in a range of 2 to 6.

Herein integer q' may be: 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

In one embodiment integer q' may be 5 or 6 or 7 or 8 or 9 or 10 or 11 or 12 or 13 or 14 or 15 or 16 or 17 or 18 or 19 or 20.

Herein, q' may be in a range of 1 to 10, for example in a range of 2 to 6.

Herein integer q" may be: 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

In one embodiment integer q" may be 5 or 6 or 7 or 8 or 9 or 10 or 11 or 12 or 13 or 14 or 15 or 16 or 17 or 18 or 19 or 20.

Herein, q" may be in a range of 1 to 10, for example in a range of 2 to 6.

Herein integer r may be: 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

Herein integer r' may be: 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

Herein integer r" may be: 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

Herein integer u may be: 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

Herein integer v may be: 1 or 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

In one embodiment integer v may be 5 or 6 or 7 or 8 or 9 or 10 or 11 or 12 or 13 or 14 or 15 or 16 or 17 or 18 or 19 or 20.

Herein, v may be in a range of 1 to 10.

Herein integer w may be: 2 or 3 or 4 or 5 or 6 or 7 or 8 or 9 or 10 or 11 or 12 or 13 or 14 or 15 or 16 or 17 or 18 or 19 or 20 or 21 or 22 or 23 or 24 or 25 or 26 or 27 or 28 or 29 or 30 or 31 or 32 or 33 or 34 or 35 or 36 or 37 or 38 or 39 or 40 or 41 or 42 or 43 or 44 or 45 or 46 or 47 or 48 or 49 or 50.

In one embodiment integer w may be 5 or 6 or 7 or 8 or 9 or 10 or 11 or 12 or 13 or 14 or 15 or 16 or 17 or 18 or 19 or 20.

Herein, w may be in a range of 2 to 10.

It will be appreciated that the above integer embodiments includes any integer range thereof. For example, a range may be provided between any two integers selected from each of the above described integer embodiments of a', b', c', d', m, m', m", n, q, q', q", r, r', r", t, u, v and w.

The copolymer segment of Formula 1 according to any of the above described embodiments may have a molecular weight range between 400 and 6000, or 400 and 4000, or 800 and 1600. Unless stated otherwise, herein the phrase "molecular weight" refers to the number-average molecular weight (Mₙ) of a particular polymer.

### Thermoplastic Polyurethane or Polyurethaneurea Elastomer Materials

Disclosed herein is a thermoplastic polyurethane or polyurethaneurea elastomer material comprising a plurality of soft segments and hard segments, wherein the plurality of soft segments are each derived from at least one copolymer segment of Formula 1 as defined herein and optionally an additional polyol or polyamine.

A thermoplastic polyurethane or polyurethaneurea elastomer material may be provided comprising a plurality of soft segments and hard segments, wherein the plurality of soft segments are each derived from a single type of block copolymer segment selected from a copolymer segment of Formula 1 as defined herein and optionally an additional polyol or polyamine.

Also disclosed herein is a thermoplastic polyurethane or polyurethaneurea elastomer material comprising a plurality of soft segments and hard segments, wherein the plurality of soft segments are each derived from a mixture of two separate copolymer segments of Formula 1 as defined herein and optionally an additional polyol or polyamine.

Also disclosed herein are thermoplastic polyurethane or polyurethaneurea elastomer materials comprising a reaction product of:
i. at least one copolymer segment of Formula 1 as defined herein;
ii. a diisocyanate;
iii. one or more chain extenders; and
iv. optionally an additional polyol or polyamine.

Herein, the thermoplastic polyurethane or polyurethaneurea elastomer material may have a tensile strength of ≥5 MPa. For example the thermoplastic polyurethane or polyurethaneurea elastomer material may have a tensile strength of: ≥ 7.5 MPa, or ≥ 10 MPa, or ≥ 12.5 MPa, or ≥ 15 MPa, or ≥ 17.5 MPa, or ≥ 20 MPa, or ≥ 22.5 MPa, or ≥ 25 MPa, or ≥ 27.5 MPa, or ≥ 30 MPa, or ≥ 32.5 MPa, or ≥ 35 MPa, or ≥ 37.5 MPa, or ≥ 40 MPa, or ≥ 42.5 MPa, or ≥ 45 MPa, or ≥ 47.5 MPa, or ≥ 50 MPa.

Herein, the thermoplastic polyurethane or polyurethaneurea elastomer material may have a Young's Modulus of ≥ 10 MPa. For example the thermoplastic polyurethane or polyurethaneurea elastomer material may have a Young's Modulus of: ≥12.5 MPa, or ≥ 15 MPa, or ≥ 17.5 MPa, or ≥ 20 MPa, or ≥ 22.5 MPa, or ≥ 25 MPa, or ≥ 27.5 MPa, or ≥ 30 MPa, or ≥ 32.5 MPa, or ≥ 35 MPa, or ≥ 37.5 MPa, or ≥ 40 MPa, or ≥ 42.5 MPa, or ≥ 45 MPa, or ≥ 47.5 MPa, or ≥ 50 MPa, or ≥ 52.5 MPa, or ≥ 55 MPa, or ≥ 57.5 MPa, or ≥ 60 MPa, or ≥ 62.5 MPa, or ≥ 65 MPa, or ≥ 67.5 MPa, or ≥ 70 MPa, or ≥ 72.5 MPa, or ≥ 75 MPa, or ≥ 77.5 MPa, or ≥ 80 MPa.

Herein, the thermoplastic polyurethane or polyurethaneurea elastomer material may have an elongation at break of ≥ 500 %. For example the thermoplastic polyurethane or polyurethaneurea elastomer material may have an elongation at break of: ≥ 550 %, or ≥ 600 %, or ≥ 650 %, or ≥ 700 %, or ≥ 750 %, or ≥ 800 %, or ≥ 850 %, or ≥ 900 %, or ≥ 950 %, or ≥ 1000 %, or ≥ 1050 %, or ≥ 1100 %, or ≥ 1150 %, or ≥ 1200 %, or ≥ 1250 %, or ≥ 1300 %, or ≥ 1350 %, or ≥ 1400 %, or ≥ 1450 %, or ≥ 1500 %, or ≥ 1550 %, or ≥ 1600 %, or ≥ 1650 %, or ≥ 1700 %, or ≥ 1750 %, or ≥ 1800 %, or ≥ 1850 %, or ≥ 1900 %, or ≥ 1950 %, or ≥ 2000 %.

It will be appreciated that these tensile strengths may be measured using standard industry methods, for example a method adopted from ASTM Standard Method for thin Plastic Sheeting (e.g. ASTM D 882-02). In a further example, the method may involve thin films, for example 200 to 300 microns, using the ASTM D 882-02 test method.

Example conditions which may be used to measure the tensile strength of a sample with ASTM D 882-02 method are:
- using dumbbell shaped specimens which are 75 mm in length, 13 mm in width at each end and 4 mm at the central narrow section (with a constant width over at least 15 mm of length); and
- using an Instron 5565 fitted with static load cell ±100 N and calibrated using Instron Bluehill 2 (version 2.35) software.

For the tensile test with dry conditions, the specimen may be:
- fixed between upper and lower grips (for example Instron grips) such that the gap between the grips is 10 mm;
- stretching the 10 mm long section of film at a rate of 50 mm per minute until the film breaks;
- performing at least three replicates, for example three, four or five replicates.

For the tensile test with wet conditions, or replicating the conditions found for medical applications, the specimen may be:
- placed in a plastic bag and immersed in water maintained at 37°C for at least two hours;
- dried using tissue paper and fixed between upper and lower grips (for example Instron grips) such that the gap between the grips is 10 mm;
- stretched at a rate of 100 mm per min until the film breaks.

Stress, strain, breaking stress and elongation at breaking can be obtained using either the dry or wet conditions from the Instron Bluehill 2 (version 2.35) software. These parameters allow one to obtain values for the tensile strength, the Young's modulus (stiffness) and Elongation (elasticity) for a sample.

Herein, the thermoplastic polyurethane or polyurethaneurea elastomer material may have a polydispersity index (PDI) of ≤ 3.00. For example the thermoplastic polyurethane or polyurethaneurea elastomer material may have a PDI of ≤ 2.50, ≤ 2.00, or ≤ 1.50.

In one embodiment a single copolymer segment of Formula 1 is used in the formulation of the thermoplastic polyurethane or polyurethaneurea elastomer material.

In another embodiment two different copolymer segments of Formula 1 are used in the formulation of the thermoplastic polyurethane or polyurethaneurea elastomer material.

Examples of mixtures one or two different copolymer segments of Formula 1 or a combination of a copolymer of Formula 1 and a macrodiol, or a copolymer segment as disclosed herein, which are used in the formulation of a thermoplastic polyurethane or polyurethaneurea elastomer material, are shown in **Table 1.** Here Segment 1 and/or Segment 2 can be added in a reaction mixture with one or more chain extenders, a diisocyanate, to produce a thermoplastic polyurethane or polyurethaneurea elastomer material. In these examples R^{5a} and R^{6a} may both be -(CH₂)₂O(CH₂)₄-.

**Table 1 - Examples of one or two different copolymer segments of Formula 1, or a combination of a copolymer of Formula 1 and a macrodiol, or a copolymer segment as disclosed herein, which are used in the formulation of a thermoplastic polyurethane or polyurethaneurea elastomer material. In the following examples, R^{5a}, R^{6a}, m, m' and r are as defined herein.**

| **Group** | **Segment Number for Block Copolymer** | **Specific Segment for Block Copolymer** |
|---|---|---|
| 1 | Segment 1 | 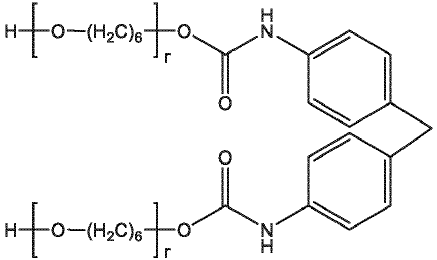 |
| | Segment 2 | - |
| 2 | Segment 1 | 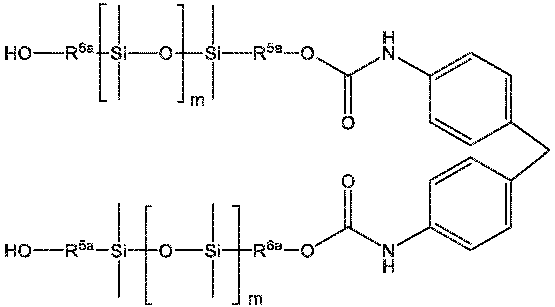 |
| | Segment 2 | - |
| 3 (Comp.) | Segment 1 | 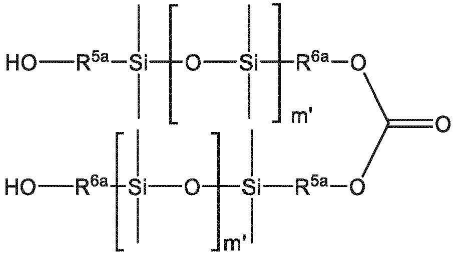 |
| | Segment 2 | |
| 4 | Segment 1 | 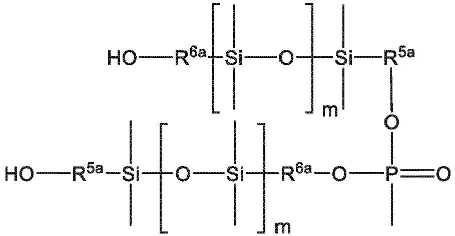 |
| | Segment 2 | - |
| 5 | Segment 1 | 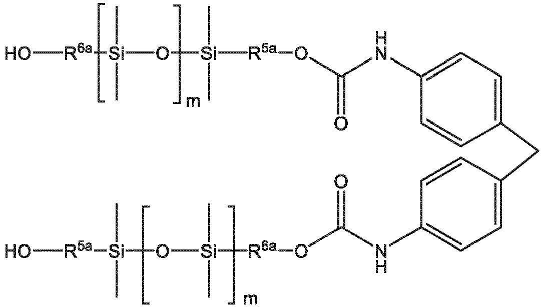 |
| | Segment 2 | 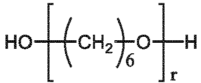 |
| 6 | Segment 1 | 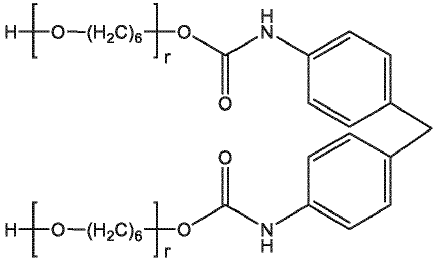 |
| | Segment 2 | 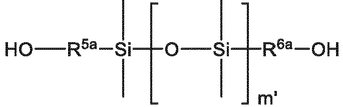 |
| 7 | Segment 1 | 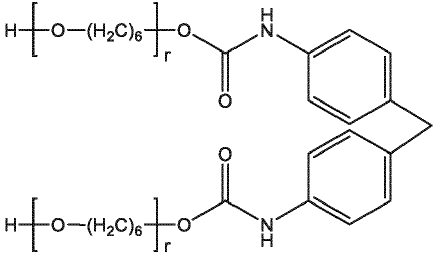 |
| | Segment 2 | 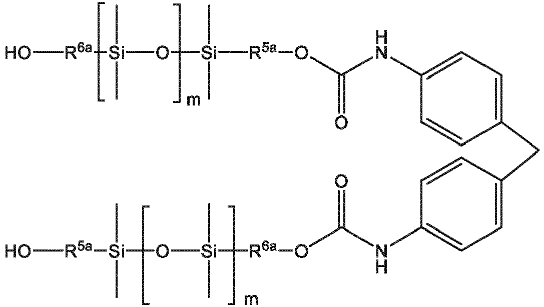 |
| 8 (Comp.) | Segment 1 | 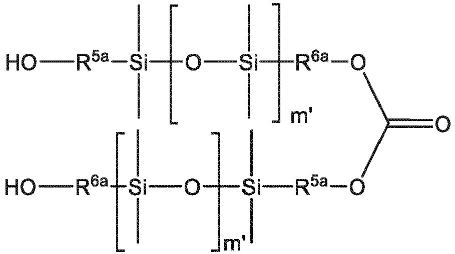 |
| | Segment 2 | 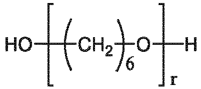 |

### Diisocyanates

Examples of appropriate diisocyanates include but are not limited to: aliphatic, cyclic or aromatic diisocyanates such as, for example: 1,4-diisocyanatobutane, 1,12-diisocyanatododecane, 1,6-diisocyantehexane, 1,8-diisocyanateoctane, 4,4'-methylenediphenyl diisocyanate (MDI), 4,4'-methylenebis(cyclohexyl diisocyanate) (H12MDI), p-phenylene diisocyanate (p-PDI), m-phenylene diisocyanate (m-PDI) trans-cyclohexane-1,4-diisocyanate (CHDI) or a mixture of the cis and trans isomers, 1,6-hexamethylene diisocyanate (HDI), 2,4-toluene diisocyanate (2,4-TDI) or its isomers (for example 2,6-toluene diisocyanate (2,6-TDI)), or mixtures thereof, p-tetramethylxylene diisocyanate (p-TMXDI), isophorone diisocyanate or m-tetramethylxylene diisocyanate (m-TMXDI), or 1,5-diisocyanatonaphthalene (NDI).

In one embodiment, the diisocyanate is MDI.

### Chain Extender

Herein at least one chain extender is included in the formation of the thermoplastic polyurethane or polyurethaneurea elastomer materials. A chain extender is a compound that has two functional groups per molecule, such as diols or diamines, which are capable of reacting with an isocyanate group.

The chain extender may have a molecular weight range of 400 or less. Alternatively, the chain extender may have a molecular weight range of 800 to 1600. In another embodiment the chain extender may have a molecular weight range of 400 to 4000.

The chain extender may be selected from diol or diamine chain extenders. In one embodiment at least one chain extender is a diol.

Examples of diol chain extenders include, but are not limited to: C₁₋₁₂alkane diols such as: 1,4-butanediol, 1,6-hexanediol, 1,8-octanediol, 1,9-nonanediol and 1,10-decanediol, 1,4-cyclohexane dimethanol, p-xyleneglycol, 1,4-bis (2-hydroxyethoxy) benzene, 1,12-dodecanediol and 1,3 bis-(4-hydroxybutyl) 1,1,3,3-tetramethyldisiloxane.

In one embodiment at least one chain extender is a diamine. Suitable diamine chain extenders include C₁₋₁₂ alkane diamines such as 1,2-ethylenediamine, 1,3-propanediamine, 1,4-butanediamine, 1,3 bis-(3-aminopropyl) tetramethyldisiloxane, or 1,3 bis-(3-aminobutyl) tetramethyldisiloxane.

The wt% of a hard segment in a thermoplastic polyurethane or polyurethaneurea (the wt% determined by the weight of a linking compound (for example a diisocyanate) + chain extender as a percentage of the total weight of the polyurethane/polyurethaneurea) may be in a range of 20 wt% to 60 wt%. Exemplified ranges include: 30 wt % to 60 wt %, or 40 wt% to 60 wt%, 50 wt% to 60 wt% and 40 wt% to 50 wt%.

In one embodiment only one chain extender is used in the formation of the thermoplastic polyurethane or polyurethaneurea elastomer material.

In one embodiment two chain extenders are used in the formation of the thermoplastic polyurethane or polyurethaneurea elastomer material. For example a combination of 1,4-butanediol and ethylene diamine.

### Additional Polyol or Polyamine

Herein a thermoplastic polyurethane or polyurethaneurea elastomer materials comprising a reaction product of: at least one copolymer segment of Formula 1 as defined herein; a diisocyanate; and one or more chain extenders, may include an additional polyol or polyamine.

The additional polyols may include poly(hexamethylene oxide), poly(heptamethylene oxide), poly(octamethylene oxide) (POMO), poly(decamethylene oxide) (PDMO), polydimethylsiloxane diols, poly(butadine diol), poly(carbonate) diol poly(isobutylene)diol, or a mixture thereof.

In one embodiment the additional polyol may be: a polydimethylsiloxane polymer comprising at least two hydroxyl groups, for example α,ω bis-(6-hydroxyethoxypropyl)polydimethylsiloxane.

In one embodiment the additional polyol is a polyol of Formula J: 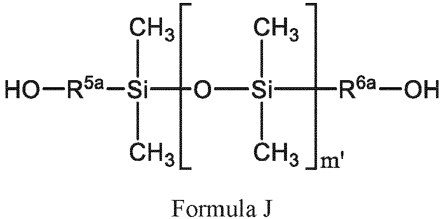 wherein:
R^{5a} and R^{6a} are each independently selected from a straight chain, branched or cyclic, saturated and unsaturated hydrocarbon radical optionally interrupted with one or more heteroatoms independently selected from O, N and S; and
m' is an integer of 1 to 50.

R^{5a} and R^{6a} may be independently selected from optionally substituted alkyl or alkoxyalkyl groups. For example R^{5a} and R^{6a} may be independently selected from optionally substituted: propylene, butylene, pentylene, hexylene, ethoxypropyl, propoxypropyl and butoxypropyl groups.

In another embodiment both of R^{5a} and R^{6a} are optionally substituted alkyl or alkoxyalkyl groups. For example R^{5a} and R^{6a} may both be selected from optionally substituted: propylene, butylene, pentylene, hexylene, ethoxypropyl, propoxypropyl and butoxypropyl groups. For example both R^{5a} and R^{6a} may be ethoxypropyl.

R^{5a} and R^{6a} may both be (CH₂)₂O(CH₂)₃.

In another embodiment the additional polyol is a polyol of Formula K': 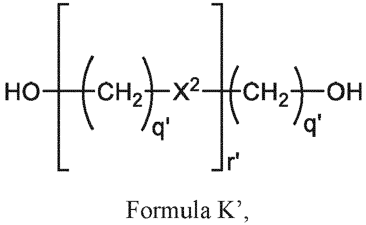 wherein:
X² is a group selected from: OC(O)O, C(O)O and O;
q' is an integer of 1 to 50, including, for example, an integer of 5 to 20; and
r' is an integer of 1 to 50.

In one embodiment X² is O.

In another embodiment the additional polyol is selected from: poly(hexamethylene oxide), poly(heptamethylene oxide), poly(octamethylene oxide) (POMO) and poly(decamethylene oxide) (PDMO).

The additional polyamines may include α,ω bis-(3-aminopropyl)polydimethylsiloxane, α,ω bis-(aminomethyl)polydimethylsiloxane, α,ω bis-(2-aminoethyl)polydimethylsiloxane, α,ω bis-(4-aminobutyl)polydimethylsiloxane, α,ω bis-(5-aminopentyl)polydimethylsiloxane and the like.

The additional polyamine may be a polyamine of Formula L: 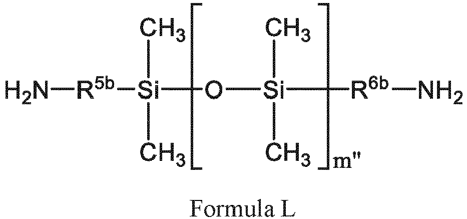 wherein:
R^{5b} and R^{6b} are each independently selected from a straight chain, branched or cyclic, saturated and unsaturated hydrocarbon radical optionally interrupted with one or more heteroatoms independently selected from O, N and S; and
m" is an integer of 1 to 50.

R^{5b} and R^{6b} may be independently selected from optionally substituted alkyl or alkoxyalkyl groups. For example R^{5b} and R^{6b} may be independently selected from optionally substituted: propylene, butylene, pentylene, hexylene, ethoxypropyl, propoxypropyl and butoxypropyl groups.

In another embodiment both of R^{5b} and R^{6b} are optionally substituted alkyl or alkoxyalkyl groups. For example R^{5b} and R^{6b} may both be selected from optionally substituted: propylene, butylene, pentylene, hexylene, ethoxypropyl, propoxypropyl and butoxypropyl groups. For example both R^{5a} and R^{6a} may be ethoxypropyl.

R^{5b} and R^{6b} may both be (CH₂)₂O(CH₂)₃.

The additional polyamine may be a polyamine of Formula M': 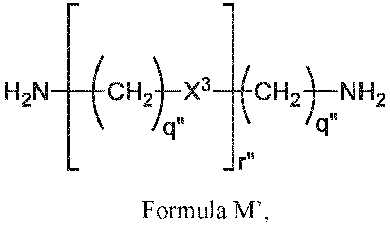 wherein:
X³ is a group selected from: OC(O)O, C(O)O and O;
q" is an integer of 1 to 50, including, for example, an integer of 5 to 20; and
r" is an integer of 1 to 50.

### PROCESSES FOR PREPARING "SOFT" BLOCK COPOLYMERS AND POLYURETHANE OR POLYURETHANEUREA ELASTOMER MATERIALS

Disclosed herein is a process for preparing a copolymer segment of Formula 1, whereby the process comprising the step of combining:
i. at least one macrodiol; or
ii. at least one macrodiamine; or
iii. a mixture of at least one macrodiol and at least one macrodiamine,
with a divalent linking compound.

Optional additional steps may include one or more of:
i. filtration;
ii. the removal of solvent(s), for example via evaporation under reduced pressure; and/or
iii. purification, for example by distillation (such as distillation via the use of Kugelrohr distillation apparatus).

Herein divalent linking compounds are compounds that can link two components, such as two macrodiols or two macrodiamines, whereby the divalent compound is at the nexus between the two components. A divalent linking compound can create: urethane, urea and phosphonate linkages within the copolymer segment.

Examples of divalent linking compounds are organophosphorus compounds or compounds comprising two functional groups, such as two isocyanate functional groups.

The divalent linking compound may be a diisocyanate, for example: 1,4-diisocyanatobutane, 1,12-diisocyanatododecane, 1,6-diisocyantehexane, 1,8-diisocyanateoctane, 4,4'-methylenediphenyl diisocyanate (MDI), 4,4'-methylenebis(cyclohexyl diisocyanate) (H12MDI), p-phenylene diisocyanate (p-PDI), m-phenylene diisocyanate (m-PDI) trans-cyclohexane-1,4-diisocyanate (CHDI) or a mixture of the cis and trans isomers, 1,6-hexamethylene diisocyanate (HDI), 2,4-toluene diisocyanate (2,4-TDI) or its isomers (for example 2,6-toluene diisocyanate (2,6-TDI)), or mixtures thereof, p-tetramethylxylene diisocyanate (p-TMXDI), isophorone diisocyanate or m-tetramethylxylene diisocyanate (m-TMXDI), 1,5-diisocyanatonaphthalene (NDI), or a mixture thereof.

The divalent compound may be an organophosphorus compound, for example methylphosphonic dichloride.

In an aspect there is provided a process for preparing a thermoplastic polyurethane or polyurethaneurea elastomer comprising the silicon based copolymer segment of Formula 1, whereby the process comprises the steps of:
i. providing a copolymer segment of Formula 1 or preparing a copolymer segment of Formula 1 using a process as described herein;
ii. optionally further reacting the material of step i. with a divalent compound and:
   a. at least one macrodiol;
   b. at least one macrodiamine; or
   c. a mixture of at least one macrodiol and at least one macrodiamine; and
iii. reacting the block copolymer segment of step i. or ii. with a chain extender or a mixture of chain extenders to form the polyurethane or polyurethaneurea elastomer.

Non-limiting examples of soft segments which could be produced for the thermoplastic polyurethane or polyurethaneurea elastomeric materials are shown in Schemes 1 to 4. In these schemes R^{5a} and R^{6a} are both (CH₂)₂O(CH₂)₄:
- Scheme 1 - The preparation of a linked macrodiol from poly(hexamethylene oxide) (PHMO) using 4,4'-methylenediphenyl diisocyanate (MDI) as the linker molecule.
- Scheme 2 - The preparation of a linked macrodiol from α,ω bis-(6-hydroxyethoxypropyl)polydimethylsiloxane (wherein R^{5a} and R^{6a} are ethoxypropyl groups) using MDI as the linker molecule.
- Scheme 3 - The preparation of a linked macrodiol also disclosed herein from α,ω-bis-(6-hydroxyethoxypropyl)polydimethylsiloxane (wherein R^{5a} and R^{6a} are ethoxypropyl groups) using a carbonate linker:
- Scheme 4 - The preparation of a linked macrodiol from α,ω-bis-(6-hydroxyethoxypropyl)polydimethylsiloxane (wherein R^{5a} and R^{6a} are ethoxypropyl groups), using a phosphonate linker.

As a non-limiting example, Scheme 5 illustrates a possible reaction scheme and the general structure of the polyurethane urea prepared from MDI linked PHMO and PDMS chain extended with ethylene diamine.

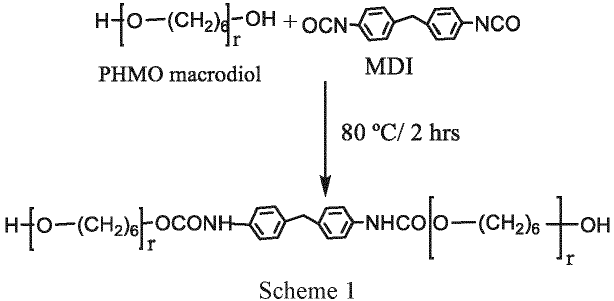

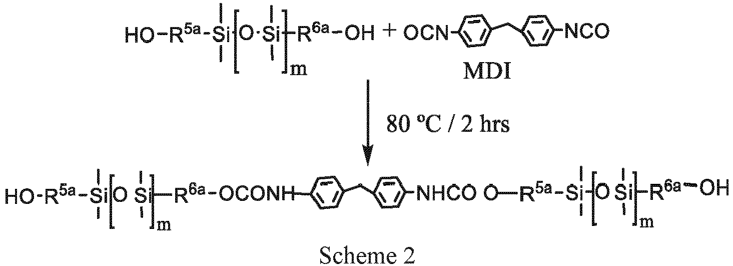

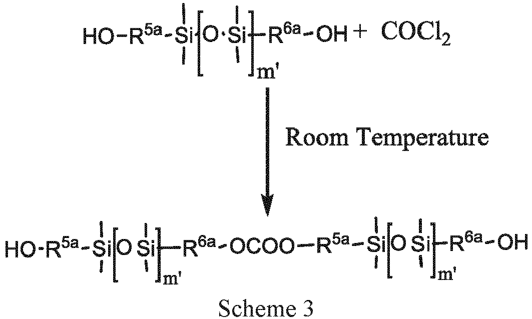

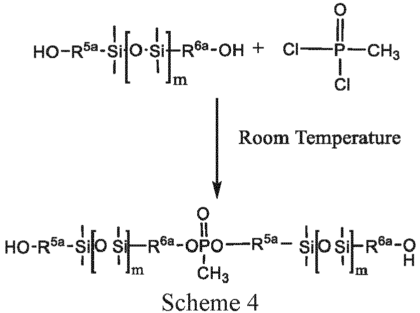

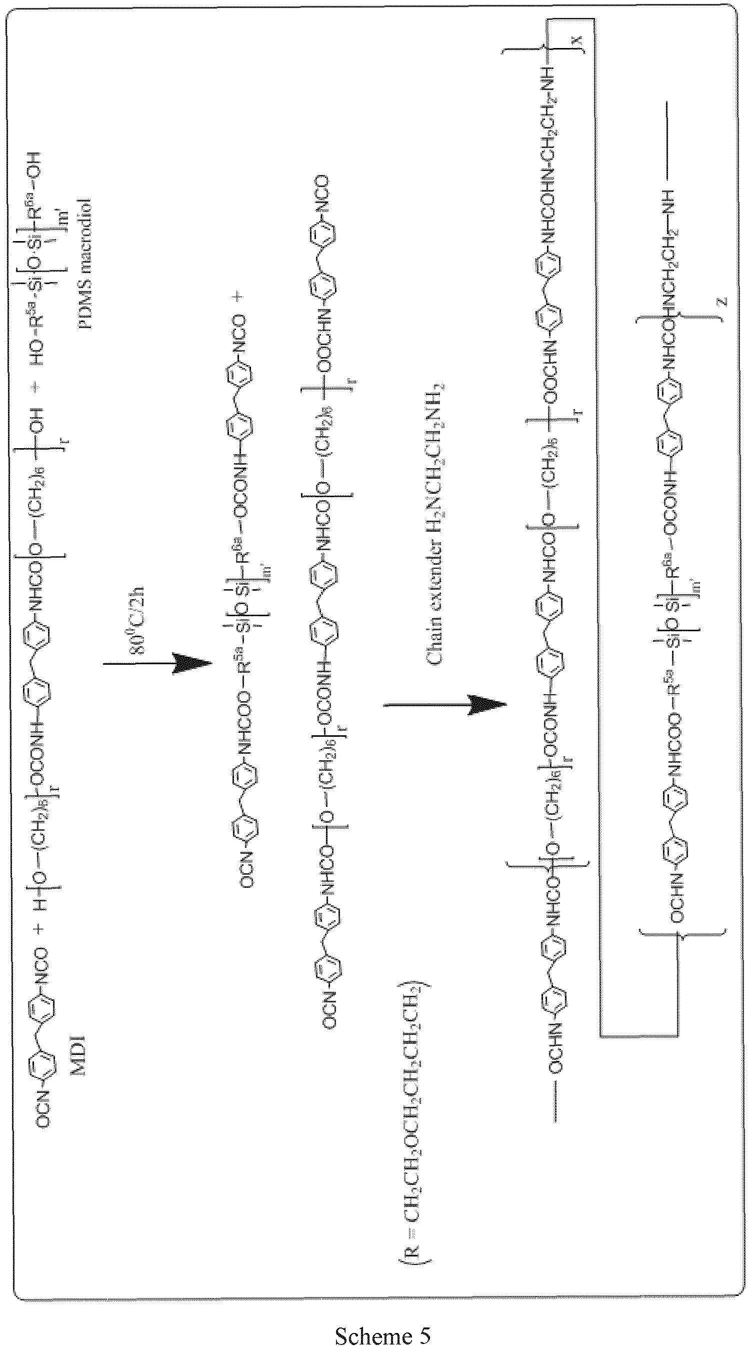

### Materials and Articles

Disclosed herein are materials that comprise a thermoplastic polyurethane or polyurethaneurea elastomer material as defined herein.

The thermoplastic polyurethane or polyurethaneurea elastomer materials may be used to produce sheets of fabrics or fibres, especially for applications where materials possessing high tensile strength and/or tear strength are required. For example the thermoplastic polyurethane or polyurethaneurea elastomer materials can be used to produce fibres that may be used in knitting or weaving speciality fabrics, for example high strength and/or water-repellent membranes. In addition, the thermoplastic polyurethane or polyurethaneurea elastomer materials may be used as sealants. For materials comprising a siloxane component, due to the dielectric properties of said siloxane component, the thermoplastic polyurethane or polyurethaneurea elastomer materials may be used for electronic and electrical components and insulation.

Also disclosed herein are articles which are composed wholly or partly of a thermoplastic polyurethane or polyurethaneurea elastomer material as defined herein.

Examples of devices or articles include: artificial leather, shoe soles, cable sheathing, varnishes, coatings, structural components for pumps or vehicles, mining ore screens, conveyor belts, laminating compounds, fibres, textiles, separation membranes, sealants, and adhesive components.

### Articles for Medical Applications

The thermoplastic polyurethane or polyurethaneurea elastomeric materials defined herein may be used as biomaterials. Herein, the term "biomaterial" is used in its broadest sense and refers to a material which is used in situations where it comes into contact with the cells and/or bodily fluids of living animals or humans.

Disclosed herein are articles which are medical devices which are composed wholly or partly of a thermoplastic polyurethane or polyurethaneurea elastomeric material as defined herein. In one embodiment the article which is a medical device is an implant.

Examples of medical devices include: a cardiac pacemaker, defibrillator, catheter, heart valve, cardiac assist device, vascular graft, implantable prosthesis, a cannula, extra-corporeal device, artificial organ, pacemaker lead, defibrillator lead, blood pump, balloon pump, A-V shunt, biosensor, membrane for cell encapsulation, drug delivery device, wound dressing, artificial joint, orthopaedic implant, or soft tissue replacement.

In one embodiment the article which is a medical device which is for example an article that relates to one or more of the aortic, mitral, pulmonary, and/or tricuspid valves.

In another embodiment the article may be a heart valve.

An example of an implantable valve 100 is presented in Fig. 1. The valve includes a plurality of valve leaflets 110-1, 110-2, and 110-3 constructed from the subject polymers. Each of leaflets 110 can be discrete from the others (as shown) or can be portions of one unitary (monolithic) leaflet body. Support structure for the valve may include an annular base portion 102 that can have a planar or flat upstream terminus or can have a curved or scalloped upstream terminus (not shown) or extensions 104 that project from annular base portion 102 downstream relative to intended flow. The leaflets can be integrally formed on this base frame 102, such as through a casting (for example dip casting) or moulding process. In an example of a dip casting process, the base frame 102 is placed on a mandrel and dipped in a polymer, which results in the formation of leaflets integrated with a polymeric coating over the base frame. In some embodiments, leaflets 110 (formed of the subject polymers) can be physically joined to support structure 102 through a coupling process such as sewing.

Herein, the thermoplastic polyurethane or polyurethaneurea elastomeric materials used in the production of a medical device should be acceptable for use on or in a recipient, such as a human being. For example, the thermoplastic polyurethane or polyurethaneurea elastomeric material, should be able to contact tissues of a recipient without excessive toxicity, irritation, allergic response or other potential complications commensurate with a reasonable benefit/risk ratio identified by a skilled medical professional or veterinarian.

It will be appreciated that in some cases thermoplastic polyurethane or polyurethaneurea elastomeric materials as described herein may not be for use on or in a recipient, such as a human being, but can be useful in the preparation of thermoplastic polyurethane or polyurethaneurea elastomeric materials which could be utilised in the preparation of materials which are acceptable for use in medical devices.

### EXAMPLES

### Raw materials

Certain chemicals referred to within the specification, including the following examples, can be obtained from the suppliers indicted in **Table 2.**

**Table 2 - Suppliers for selected compounds disclosed in the examples.**

| **Component** | **Example Supplier** |
|---|---|
| α,ω Bis-(6-hydroxyethoxypropyl)pol ydimethylsiloxane (molecular weight 928) | Shin-Etsu |
| Hydrogenated poly(butadiene) diol | Kraysol® from Cray Valley |
| 4,4'-methylene diphenyl diisocyanate | BASF |
| 1,4-Butanediol | Aldrich |
| 1,3 bis-(3-aminopropyl) tetramethyldisiloxane | Shin-Etsu |
| 1,1,3,3-Bis-hydroxybutyl tetramethyl disiloxane | Shin-Etsu |

### Equipment

### Gel Permeation Chromatography: Waters THF System

Gel permeation chromatography (GPC) was performed on a Waters Alliance system equipped with an Alliance 2695 Separations Module (integrated quaternary solvent delivery, solvent degasser and autosampler system), a Waters column heater module, a Waters 2414 RDI refractive index detector, a Waters PDA 2996 photodiode array detector (210 to 400 nm at 1.2nm) and 4× Agilent PL-Gel columns (3 x PL-Gel Mixed C (5µm) and 1 x PL-Gel Mixed E (3 µm) columns), each 300 mm × 7.8 mm², providing an effective molar mass range of 10 to 4 × 10⁵). Tetrahydrofuran (THF) high purity solvent (HPLC grade)was pre-filtered through aluminium oxide (90 active neutral, 70-230 mesh) with 0.45 µm filter, and 0.1 g L⁻¹ *2*,*6*-di-tert-butyl-*4*-methylphenol (BHT) was added as inhibitor. The filtered THF containing BHT was purged slowly with nitrogen gas and used as an eluent with a flow rate of 1 mL/min at 30°C. Number (*M*ₙ) and weight average (*M*_{w}) molar masses were evaluated using Waters Empower-3 software. The GPC columns were calibrated with low dispersity polystyrene (PSt) standards (Polymer Laboratories) ranging from 265 to 2,560,000 g mol⁻¹, and molar masses are reported as PSt equivalents. A 3^{rd}-order polynomial was used to fit the log *M*ₚ *vs.* time calibration curve, which was near linear across the molar mass ranges.

### Gel Permeation Chromatography: Shimadzu - DMAc

Gel permeation chromatography (GPC) was performed on a Shimadzu system equipped with a CMB-20A controller system, an SIL-20A HT autosampler, an LC-20AT tandem pump system, a DGU-20A degasser unit, a CTO-20AC column oven, an RDI-10A refractive index detector, and 4 × Waters Styragel columns (HT2, HT3, HT4, and HT5, each 300 mm × 7.8 mm², providing an effective molar mass range of 100-4 × 10⁶). *N*,*N*-Dimethylacetamide (DMAc) (containing 4.34 g L⁻¹ lithium bromide (LiBr)) was used as an eluent with a flow rate of 1 mL/min at 80 °C. Number (*M*ₙ) and weight average (*M*_{w}) molar masses were evaluated using Shimadzu LC Solution software. The GPC columns were calibrated with low dispersity polystyrene (PSt) standards (Polymer Laboratories) ranging from 575 to 3,242,000 g mol⁻¹, and molar masses are reported as PSt equivalents. A 3^{rd}-order polynomial was used to fit the log *M*ₚ *vs.* time calibration curve, which was near linear across the molar mass ranges

### Film preparation by solvent casting and testing of mechanical properties:

Polymer films of 80 mm x 145 mm size were prepared by placing a solution of the polymer in a Teflon mould and then evaporating the solvent slowly in a nitrogen circulating oven at 60 °C for few hours followed by further drying under a vacuum (1 torr) overnight. The films were then equilibrated at room temperature for at least 24 hours before using them for tensile property measurement.

Dumbbell shaped specimens of polymer film were punched using a die and a manual cutting Press (IDM Instruments). The specimens had dimensions of 75 mm length, 13 mm width at each end and 4 mm at the central narrow section (constant width over at least 15mm of length).Thickness of the cut out specimen was measured using a digital thickness gauge (Mitutoyo, Japan). In case there was small variation in thickness over the length of 15 mm in the central narrow section, an average of three thickness values was taken. Instron 5565 fitted with static load cell ±100 N was initialized and the load was calibrated using Instron Bluehill 2 (version 2.35) software. For the tensile test at dry condition, the specimen was fixed between upper and lower grips (Instron) such that the gap between the grips was 10 mm. The 10 mm long section of the film was stretched at the rate of 50 mm per minute until the film broke. At least three replicates of specimen were tested for each film. In case of wide discrepancy between results, five tests were carried out. Stress, strain, breaking stress and elongation at breaking were obtained from the software. These parameters allow one to obtain values for the tensile strength, the Young's modulus (stiffness) and Elongation (elasticity) of the samples.

For the tensile test at wet condition, (or replication of conditions found in medical applications) the cut out specimen of known thickness was put in a plastic bag and immersed in water maintained at 37 °C for at least two hours. The specimen was quickly dried using tissue paper and fixed between the grips and stretched at the rate of 100 mm per min until the film broke. Stress, strain, breaking stress and elongation at breaking were obtained from the software. The stress values were plotted against strain (% of initial value).

### Abbreviated Terms

**Table 3** lists a series of abbreviated terms which are used herein.

**Table 3 - Acronyms used for compounds and components described herein**

| **Acronym** | **Compound/Component** |
|---|---|
| BAPD | 1,3 Bis-(3-aminopropyl) tetramethyldisiloxane |
| DMAc | N'N-Dimethylacetamide |
| GPC | Gel Permeation Chromatography |
| MDI | 4,4' -Methylenediphenyldiisocyanate |
| Mₙ | Number Average Molecular Weight |
| M_{w} | Weight Average Molecular Weight |
| PDI | Polydispersity Index |
| PDMS | α,ω Bis-(6-hydroxyethoxypropyl)polydimethylsiloxane |
| PHMO | Poly(hexamethylene oxide) |
| PU | Polyurethane |
| PUU | Polyurethaneurea |
| EDA | 1,2-ethanediamine |
| BDO | 1,4-butanediol |
| BHTD | 1,1,3,3-bis-hydroxybutyltetramethylene disiloxane |

### Example 1 - Synthesis of Urethane Linked Poly(hexamethylene oxide) (PHMO-u-PHMO)

Poly(hexamethylene oxide) (PHMO) (molecular weight 696.23) was prepared according to a method described in:
- P. A. Gunatillake, G. F. Meijs, R. C. Chatellier, D. M. McIntosh and E. Rizzardo, Polym. Int., 7, 275-283, 1992; and
- US patent No. 5403912.
The PHMO was dried and degassed by heating at 105 °C for about 15 hours under vacuum (0.1 torr) until the moisture content was below 200 ppm, as determined by Karl Fisher titration. All glassware used were dried overnight at 105 °C prior to use in the experiment. Accurately weighed molten 4,4'-methylenediphenyldiisocyanate (MDI) (7.18 g) was placed in a round bottom flask equipped with: a mechanical stirrer, addition funnel and a nitrogen inlet. The flask was then placed in an oil batch at 80 °C. Pre-dried PHMO (40.00 g) was weighed and added to MDI with stirring over a period of 20 minutes. The reaction mixture was further reacted for about 2 hours until all the isocyanate was consumed. This was confirmed by the absence of an IR absorption band at 2275 cm⁻¹.

Characterisation data for the PHMO prior to and after linking is shown in **Table 4**.

**Table 4 - Molecular Weight Characterisation Data for Example 1:**

| **System** | **Mₙ** | **M_{w}** | **PDI** |
|---|---|---|---|
| PHMO - Before Linking | 1333 | 1999 | 1.49 |
| PHMO - After Linking (Urethane) | 4322 | 7272 | 1.68 |

The linked-PHMO was stored under nitrogen at ambient temperature until further use.

### Example 2 Synthesis of Urethane Linked α,ω-Bis-(6-hydroxyethoxypropyl)polydimethylsiloxane (PDMS-u-PDMS)

α,ω-Bis-(6-hydroxyethoxypropyl)polydimethylsiloxane (molecular weight 928) was dried and degassed at 105 °C for about 15 hours until the moisture content was below 200 ppm as determined by Karl Fisher titration. All glassware used were dried overnight at 105 °C before use in the experiment. Accurately weighed molten MDI (6.74 g) was placed in a round bottom flask equipped with: a mechanical stirrer, addition funnel and a nitrogen inlet. The flask was then placed in an oil batch at 80 °C. Pre-dried PDMS (50.0 g) was weighed accurately and added to MDI with stirring over a period of 20 minutes. The reaction mixture was further reacted with stirring at 80 °C for about 2 hours until all the isocyanate was consumed, which was confirmed by the absence of an IR absorption band at 2275 cm⁻¹.

Characterisation data for the PHMO prior to and after linking is shown in **Table 5**.

**Table 5 - Molecular Weight Characterisation Data for Example 2:**

| **System** | **Mₙ** | **M_{w}** | **PDI (=M_{w}/Mₙ)** |
|---|---|---|---|
| Siloxane - Before Linking | 1334 | 1475 | 1.30 |
| Siloxane - After Linking (Urethane) | 5302 | 7468 | 1.40 |

The results showed that the linking reaction was successful. Following the reactions, the product was stored under nitrogen at ambient temperature.

### Compararitve Example 3 - Synthesis of the carbonate linked PDMS (PDMS-c-PDMS)

In a three-necked 1L round bottomed flask equipped with: a silica gel drying tube, a 250 mL pressure compensating dropping funnel, a thermometer, and a magnetic stirrer bar, were placed 200 ml of dry toluene and 49.48g of a 20% solution of phosgene in toluene. The flask was cooled, whilst stirring, to 0 °C. 23.62 g of pyridine was added dropwise to the mixture from the dropping funnel. The addition was made over a 15 minute period during which time the temperature was maintained in the range 0-5 °C. 270.82 g of α,ω-bis(hydroxyethoxypropyl) polydimethylsiloxane (molecular weight 928) was then added drop wise to the mixture from the dropping funnel. The addition was made over a 1 hour period during which time the temperature of the mixture rose to 15 °C after which the reaction mixture was maintained at 15 °C for 2 hours. During this period pyridine hydrochloride precipitated out of the reaction mixture. The reaction mixture was filtered through celite to remove the pyridine hydrochloride. The toluene was removed by rotary evaporator at 80 °C under a reduced pressure of 20 torr. The mixture was transferred to a Kugelrohr distillation apparatus and stripped of low molecular weight species at 150 °C under a reduced of 8 × 10⁻² torr to give 202 g of (PDMS-1000)-c-(PDMS-1000) as a colourless oil (molecular weight 1924).

### Example 4 - Synthesis of the Phosphate linked PDMS (PDMS-p-PDMS)

In a 500 ml round bottomed flask equipped with a magnetic stirrer bar were placed 100 ml of dry ether, 69.82 g of α,ω-bis(hydroxyethoxypropyl) polydimethylsiloxane (molecular weight 928), and 7.61 g of triethylamine. A solution of 23.62 g of methylphosphonic dichloride in 50 ml of ether was added drop wise to the mixture from the dropping funnel. During this period triethylamine hydrochloride precipitated out of the reaction mixture. The reaction was stirred for a further 3 days at room temperature. The reaction mixture was filtered to remove the pyridine hydrochloride. The ether was removed by rotary evaporator at 80 °C under a reduced pressure of 20 torr to give 72 g of PDMS-1000-P(O)Me-PDMS as a colourless oil.

### Example 5 - Preparation of a polyurethaneurea (PUU) using linked PHMO-u-PHMO, PDMS, BAPD with 40 hard segment percentage

Accurately weighed linked PHMO-u-PHMO (10.00 g) prepared according to the method described in Example 1, and α,ω-bis(hydroxyethoxypropyl) polydimethylsiloxane (PDMS) (MW 973, 40.00 g) were mixed in a flask and degassed at 80 °C under vacuum (0.1 torr) for 2 hours. MDI (22.94 g) was accurately weighed in to a round bottom flask equipped with: a mechanical stirrer, addition funnel and a nitrogen inlet. The flask was then placed in an oil batch at 80 °C. The mixture of PHMO-u-PHMO and PDMS was added slowly over period of 30 minutes using an addition funnel to with stirring to MDI in the flask. After the addition was over, the reaction mixture was heated at 80 °C for 2 hours with stirring under nitrogen. Anhydrous N'N-dimethylacetamide (DMAc, 500 mL) was then added using a syringe to the reaction mixture and stirred for 5 minutes until a clear solution is obtained. The solution was cooled in an ice bath to 0 °C and 1,3 bis-(3-aminopropyl) tetramethyldisiloxane (BAPD, 10.40 g) dissolved in anhydrous DMAc (25 mL) was added dropwise to the pre-polymer solution in the flask with stirring. After the addition was over, the polymer solution was heated to 90 °C for a period of 3 hours and transferred into a Schott bottle.

The polymer solution, after allowing to degas, was cast as thin film by pouring on to a Teflon mould. The mould was placed in an oven under a slow stream of nitrogen at 60 °C for 6 hours to remove the solvent, followed by placing it under vacuum (0.1 torr) for a further 16 hours to remove any remaining solvent.

Characterisation data for the prepared polymer is shown in **Table 6**.

### Example 6 - Preparation of a polyurethaneurea (PUU) using linked PDMS-u-PDMS, PHMO, BAPD with 40 hard segment percentage

Accurately weighed linked PDMS-u-PDMS (40.00 g) prepared according to the method described in Example 2, and PHMO (MW 696, 10.00 g) were mixed in a flask and degassed at 80 °C under vacuum (0.1 torr) for 2 hours. MDI (21.19 g) was accurately weighed in to a round bottom flask equipped with: a mechanical stirrer, addition funnel and a nitrogen inlet. The flask was then placed in an oil batch at 80 °C. The mixture of PDMS-u-PDMS and PHMO was added slowly over period of 30 minutes using an addition funnel to with stirring to MDI in the flask. After the addition was over, the reaction mixture was heated at 80 °C for 2 hours with stirring under nitrogen. Anhydrous DMAc (500 ml) was then added using a syringe to the reaction mixture and then stirred for 5 minutes until a clear solution was obtained. The solution was cooled in an ice bath to 0 °C and BAPD (12.14 g) dissolved in anhydrous DMAc (25 mL) was added dropwise to the pre-polymer solution in the flask with stirring. After the addition was over, the polymer solution was heated to 90 °C for a period of 3 hours and transferred into a Schott bottle.

The polymer solution, after allowing to degas, was cast as thin film by pouring on to Teflon mould. The mould was placed in an oven under a slow stream of nitrogen at 60 °C for 6 hours to remove the solvent, followed by placing the mould under vacuum (0.1 torr) for 16 hours to remove any remaining solvent.

Characterisation data for the prepared polymer is shown in **Table 6**.

### Comparative Example 7 - Preparation of a polyurethaneurea (PUU) using linked PDMS-c-PDMS, PHMO, BAPD with 40 hard segment percentage

Accurately weighed linked PDMS-c-PDMS (40.00 g) prepared according to the method described in Comparative Example 3, and PHMO (molecular weight 696, 10.00 g) were mixed in a flask and degassed at 80 °C under vacuum (0.1 torr) for 2 hours. MDI (21.42 g) was accurately weighed in to a round bottom flask equipped with: a mechanical stirrer, addition funnel and a nitrogen inlet. The flask was then placed in an oil batch at 80 °C. The mixture of PDMS-c-PDMS and PHMO was added slowly over period of 30 minutes using an addition funnel to with stirring to MDI in the flask. After the addition was over, the reaction mixture was heated at 80 °C for 2 hours with stirring under nitrogen. Anhydrous DMAc (500 mL) was then added using a syringe to the reaction mixture and stirred for 5 minutes until a clear solution was obtained. The solution was cooled in an ice bath to 0 °C and BAPD (11.91 g) dissolved in anhydrous DMAc (25mL) was added dropwise to the pre-polymer solution in the flask with stirring. After the addition was over, the polymer solution was heated to 90 °C for a period of 3 hours and transferred into a Schott bottle.

The polymer solution, after allowing to degas, was cast as thin film by pouring on to Teflon mould. The mould was placed in an oven under a slow stream of nitrogen at 60 °C for 6 hours to remove DMAc, followed by under vacuum (0.1 torr) for 16 hours to remove any remaining solvent.

Characterisation data for the prepared polymer is shown in **Table 6**.

### Example 8 - Preparation of a polyurethaneurea (PUU) using linked PDMS-u-PDMS and BAPD without PHMO with 40 hard segment percentage

Accurately weighed linked PDMS (40.0 g) was degassed at 80 °C for 2 hours under vacuum (0.1 torr). Molten MDI (15.88 g) was placed in a three necked flask equipped with: a mechanical stirrer, dropping funnel and a nitrogen inlet. The flask was then placed in an oil bath at 70 °C. The degassed macrodiol mixture (50 g) was added dropwise through the addition funnel over a period of 30 minutes. After the addition was over, the reaction mixture was heated at 80 °C for 2 hours with stirring under nitrogen. Anhydrous DMAc (500 mL) was then added through a syringe to the reaction mixture and stirred for 5 minutes until it was a clear solution. The solution was then cooled in an ice bath to 0 °C and BAPD (10.79 g) mixed with anhydrous DMAc (50 mL) was added dropwise into the above solution. After the addition was over, the above polymer solution was heated to 90 °C for a period of 3 hours and transferred into a Schott bottle. The polymer solution was then degassed at 60 °C in a nitrogen circulating oven and cast into a 0.5-mm thick film.

The polymer solution, after allowing to degas, was cast as thin film by pouring on to Teflon mould. The mould was placed in an oven under a slow stream of nitrogen at 60 °C for 6 hours to remove solvent. The mould was then placed under vacuum (0.1 torr) for 16 hours to remove any remaining solvent.

### Comparative Example 9 - Preparation of a polyurethaneurea (PUU) using linked PDMS-c-PDMSO, BDO with 40 hard segment percentage by one-step bulk polymerisation

A mixture of pre-dried linked PDMS-c-PDMS (20.0 g) and 1.4-butanediol (BDO) (2.85 g) was weighed in a PP beaker and degassed at 80 °C under a vacuum of 0.1 torr for over an hour. The vacuum was released under nitrogen and dibutyltindilaurate (DBTL) catalyst 0.1 wt-% was added and stirred manually with the spatula. Molten MDI (10.49g) was then added to the reaction mixture at once and stirred rapidly using spatula until viscous enough and then poured into Teflon tray and cured at 100 °C for 18 hours. The polymer was then compression moulded into a film using hot press at 180 °C for mechanical properties.

### Example 10 - Preparation of a polyurethaneurea (PUU) using linked PHMO-u-PHMO, PDMS, BAPD with 50 hard segment percentage

A PUU was synthesised according procedure as described in Example 5. The following quantities of reagents were used:
- linked PHMO-u-PHMO (10.0g);
- PDMS (40.0 g);
- MDI (31.68 g); and
- BAPD (18.32 g).

Characterisation data for the prepared PUU is shown in **Table 6**.

### Example 11 - Preparation of a polyurethaneurea (PUU) using linked PHMO-u-PHMO, PDMS, EDA with 50 hard segment percentage

The PUU was synthesised according procedure as described in Example 5. The following quantities of reagents were used:
- linked PHMO-u-PHMO (10.0 g);
- PDMS (40.0 g);
- MDI (42.94 g); and
- EDA (7.06) g.

Characterisation data for the prepared PUU is shown in **Table 6**.

### Example 12 - Preparation of a polyurethaneurea (PUU) using linked PDMS-u-PDMS, PHMO, BAPD with 50 hard segment percentage

A PUU was synthesised according to the procedure as described in Example 5. The following quantities of reagents were used:
- linked PDMS-u-PDMS (40.0 g);
- PHMO (10.0 g);
- MDI (29.39 g); and
- BAPD (20.61 g).

Characterisation data for the prepared PUU is shown in **Table 6**.

### Example 13 - Preparation of a polyurethaneurea (PUU) using linked PHMO-u-PHMO, PDMS, BAPD:EDA (8:2) with 50 hard segment percentage

A mixture of PDMS (40.0g) and PHMO-u-PHMO (10.0 g) was degassed at 80 °C for 2 hours under vacuum (0.1 torr). Molten MDI (32.91 g) was placed in a three necked flask equipped with: a mechanical stirrer, dropping funnel and a nitrogen inlet. The flask was then placed in an oil bath at 70 °C. The degassed macrodiol mixture (50 g) was added dropwise through the addition funnel over a period of 30 minutes. After the addition was over, the reaction mixture was heated at 80 °C for 2 hours with stirring under nitrogen. Anhydrous DMAc (500 mL) was then added through a syringe to the reaction mixture and stirred for 5 minutes until it was a clear solution. The solution was then cooled in an ice bath to 0 °C. BAPD (16.11 g) mixed with ethylene diamine (0.97 g) dissolved in anhydrous DMAc (60 mL) was added dropwise into above solution. After the addition was over, the above polymer solution was heated to 90 °C for a period of 3 hours and transferred into a Schott bottle. The polymer solution was then degassed at 60 °C in a nitrogen circulating oven and cast into a thin film as described in Example 5.

Characterisation data for the prepared PUU is shown in **Table 6**.

### Example 14 - Preparation of a polyurethaneurea (PUU) using linked PHMO-u-PHMO, PDMS, BHTD:EDA (6:4) with 45 hard segment percentage

A mixture of PDMS (40.0g) and PHMO-u-PHMO (10.0 g) was degassed at 80 °C for 2 hours under vacuum (0.1 torr). Molten MDI (28.65 g) was placed in a three necked flask equipped with: a mechanical stirrer, dropping funnel and a nitrogen inlet. The flask was then placed in an oil bath at 70 °C. The degassed macrodiol mixture (50.0g) was added through the addition funnel over a period of 30 minutes. After the addition was over, the reaction mixture was heated at 80 °C for 2 hours with stirring under nitrogen. 1,1,3,3-Bis-hydroxybutyltetramethylene disiloxane (BHTD) (10.72g) was then added to the reaction mixture and the system was allowed to react for a further 2 hours. The reaction mixture was then cooled down to 0 °C and anhydrous DMAc (500 mL) was then added through a syringe to the reaction mixture and stirred for 5 minutes until it was a clear solution. EDA (1.54 g) dissolved in anhydrous DMAc (60 mL) was added dropwise into above solution. After the addition was over, the above polymer solution was heated to 90 °C for a period of 3 hours and then transferred into a Schott bottle. The polymer solution was then degassed at 60 °C in a nitrogen circulating oven and cast into a thin film as described in Example 5.

Characterisation data for the prepared PUU is shown in **Table 6**.

### Example 15 - Preparation of a polyurethaneurea (PUU) using linked PHMO-u-PHMO, PDMS, BHTD:EDA (4:6) with 45 hard segment percentage

The PUU was synthesised according to the procedure described in Example 14. The following quantities of reagents were used:
- linked PHMO-u-PHMO (10.0g);
- PDMS (40.0 g);
- MDI (30.42 g);
- BHTD (7.93 g); and
- EDA (2.57 g).

Characterisation data for the prepared PUU is shown in **Table 6**.

### Example 16 - Preparation of a polyurethaneurea (PUU) using linked PHMO-u-PHMO, PDMS, BDO:EDA (6:4) with 45 hard segment percentage

The PUU was synthesised according to the procedure described in Example 14. The following quantities of reagents were used:
- linked PHMO-u-PHMO (10.0 g);
- PDMS (40.0 g);
- MDI (34.19 g);
- BHTD (4.65 g); and
- BDO (2.07 g).

Characterisation data for the prepared PUU is shown in **Table 6**.

### Example 17 - Preparation of a polyurethaneurea (PUU) using linked PDMS-u-PDMS, PHMO, BHTD:EDA (6:4) with 45 hard segment percentage

The PUU was synthesised following procedure as described in Example 14. The amount of precursors used are as follows:
- linked PHMO-u-PHMO (10.0 g);
- PDMS (40.0 g);
- MDI (26.92 g);
- BHTD (12.23 g); and
- EDA (1.76 g).

Characterisation data for the prepared PUU is shown in **Table 6**.

### Example 18 - Preparation of a polyurethaneurea (PUU) using linked PDMS-u-PDMS, PHMO-u-PHMO, BHTD:EDA (6:4) with 45 hard segment percentage

The PUU was synthesised according to the procedure described in Example 14. The following quantities of reagents were used:
- linked PHMO-u-PHMO (10.0 g);
- PDMS-u-PDMS (40.0 g);
- MDI (26.02 g);
- BHTD (13.02 g); and
- EDA (1.87 g).

Characterisation data for the prepared PUU is shown in **Table 6**.

### Example 19 - Preparation of a polyurethaneurea (PUU) using linked PHMO-u-PHMO, PDMS, BHTD:EDA (50:50) with 45 hard segment weight percentage

The PUU was synthesised according to the procedure described in Example 14. The following quantities of reagents were used:
- linked PHMO-u-PHMO (20.0g);
- PDMS (80.0 g);
- MDI (58.33 g);
- BHTD (19.32 g); and
- EDA (4.17 g).

### Example 20 - Preparation of a polyurethaneurea (PUU) using linked PHMO-u-PHMO, PDMS, BAPD with 40 hard segment weight percentage

The PUU was synthesised according to the procedure described in Example 14. The following quantities of reagents were used:
- linked PHMO-u-PHMO (10.0g);
- PDMS (40.0 g);
- MDI (29.32 g); and
- BAPD (4.02 g).

### Example 21 - Tensile testing of the materials produced in Examples and Comparative Examples 5-7, 10 & 12-20.

Tensile testing was carried out using dumbbells punched from dried polyurethaneurea films prepared in Examples and Comparative Examples 5 to 7, 10 and 12-20. Tensile testing was carried out on an Instron model 5565 Universal Testing Machine. The results are summarised in **Table 6**. These results indicate that the polyurethaneurea polymers of the current invention demonstrate high tensile strength and high elongation compared to polyurethaneurea polymers in the prior art.

Tensile testing of films prepared in Examples 13, 17, 18 and 20 was carried out on an Instron model 5565 Universal Testing Machine using the procedure described under the heading "Equipment".

Characterisation data for the prepared PUUs are shown in **Table 6** and **Table 7**.

**Table 6 - The molecular weight and tensile properties of polyurethaneureas prepared as described in Examples and Comparative Examples 5-7, 10 and 12-20 measured at ambient temperature.**

| **Example** | **Ultimate Tensile Strength (MPa)** | **Young's Modulus (MPa)** | **Elongation at Break (%)** | **GPC Data** | | |
|---|---|---|---|---|---|---|
| | | | | **Mₙ** | **Mw** | **PDI** |
| 5 | 20.6 ± 2.5 | 11.20 ± 0.8 | 1871 ± 26 | 85762 | 192726 | 2.2 |
| 6 | 22.26 ± 1.7 | 16.6 ± 0.7 | 1361± 12 | 86486 | 208711 | 2.35 |
| Comp. 7 | 6.0 ± 0.8 | 20.7 ± 4.0 | 570 ± 20 | 69106 | 214173 | 2.8 |
| 10 | 14.98 ± 6.3 | 16.6 ± 0.7 | 1361 ± 12 | 72816 | 142744 | 1.94 |
| 12 | 20.6 ± 2.5 | 11.20 ± 0.8 | 1871 ± 26 | 85762 | 192726 | 2.2 |
| 13 | 28.47 ± 1.5 | 57.7 ± 2.6 | 830 ± 4.9 | 119896 | 351073 | 2.53 |
| 14 | 18.5 ± 3.2 | 21.6 ± 3.0 | 1170 ± 13 | 143469 | 544021 | 2.73 |
| 15 | 36.8 ± 0.8 | 52.8 ± 0.5 | 1100 ± 30 | 129302 | 272731 | 2.01 |
| 16 | 31.7 ± 0.3 | 66.2 ± 6.1 | 860 ± 84 | 82800 | 156265 | 1.83 |
| 17 | 26.6± 13.4 | 39.28± 8.5 | 990 ± 36 | 123109 | 394717 | 2.65 |
| 18 | 36.7 ± 3.6 | 35.1 ± 1.8 | 1180 ± 11 | 104911 | 213576 | 1.95 |
| 19 | 34.0 ±2.7 | 35.8 ± 2.1 | 1367 ± 42 | 89147 | 253274 | 2.84 |
| 20 | 23.6 ±2.4 | 67.97 ± 10.9 | 800 ± 12 | 87372 | 158008 | 1.73 |

**Table 7 - The tensile properties of polyurethaneureas prepared as described in Examples 13, 17, 18, and 20 measured at 37 °C temperature.**

| **Example** | **Ultimate Tensile Strength (MPa)** | **Young's Modulus (MPa)** | **Elongation at Break (%)** |
|---|---|---|---|
| 13 | 27.53 ± 2.8 | 29.44 ± 0.63 | 950 ± 3 |
| 17 | 22.96 ± 10.64 | 37.09 ± 4.8 | 860 ± 25 |
| 18 | 30.55 ± 3.2 | 28.84 ± 0.59 | 1180 ± 11 |
| 20 | 23.06 ± 2.3 | 56.49 ± 4.2 | 880 ± 5 |

The polyurethaneureas prepared according to the present invention exhibit high tensile strength, low modulus and high elongation as shown in Table 6. In particular, the polyurethaneureas prepared according to Examples 15 and 16 had tensile strength exceeding 30 MPa with elongation at break over 1000%. This combination of properties is unique and far exceeded those reported in prior art. For example, the polyurethaneureas disclosed in International Patent Application WO00/64971 had elongations at break less than 500% for materials described in all Examples.

### Example 22: Preparation of polyurethaneureas using linked-PHMO and linked PDMS synthesized from different diisocyanates

Diisocyanates MDI, 1,6 hexamethylene diisocyanate (HDI) and isophorone diisocyanate (IPDI) were used as linker diioscyanates in preparing series of PUUs in this Example.

PHMO and PDMS were purified as described in Example 1 and 2, respectively. Chain extenders 1,2 ethylenediamine (EDA) and 1,3-bis(4-hydroxybutyl)-1,1,3,3-tetramethyldisiloxane (BHTD), received respectively from Aldrich and Silar Lab were used as received.

### Preparation of Linked Macrodiols:

100.0 g of pre-dried PHMO (molecular weigh 713 g·mol⁻¹) was placed in a three-neck round-bottom flask equipped with a magnetic stirrer and a nitrogen inlet. The flask was placed in an oil bath at 80 °C. 17.53 g of molten MDI (molecular weight 250.25 g·mol⁻¹) was placed in an addition funnel and added to PHMO while stirring within one minute. The reaction mixture was further reacted for two hours and the completion of the reaction was monitored using Fourier transform infrared (FTIR) spectroscopy.

Similarly, PDMS (molecular weight 998 g·mol⁻¹) was linked with MDI. A similar procedure was followed to link PHMO and PDMS with HDI and IPDI separately. Amounts of the compounds used for each macrodiol coupling is tabulated in Table 8. All the linked macrodiols were degassed at 80 °C under vacuum (0.1 torr) for 15 hours prior to use in experiments. Molecular weights were determined by the hydroxyl number determination and were in agreement with the theoretical values.

**Table 8 - Weight of macrodiol and diisocyanate used in preparing linked-macrodiols**

| **Linked-Macrodiol** | **Weight /g** | |
|---|---|---|
| | **Macrodiol** | **Diisocyanate** |
| PHMO-MDI-PHMO | 100 | 17.53 |
| PDMS-MDI-PDMS | 150 | 18.80 |
| PHMO-HDI-PHMO | 100 | 11.54 |
| PDMS-HDI -PDMS | 100 | 8.42 |
| PHMO-IPDI-PHMO | 100 | 15.24 |
| PDMS-IPDI-PDMS | 100 | 11.13 |

### Preparation of Polyurethenureas:

Molten MDI (7.29 g) was weighed into a three-neck round-bottom flask equipped with a mechanical stirrer and nitrogen inlet. The flask was place in an oil bath at 70°C. A mixture of pre-dried linked PHMO-MDI-PHMO (2.5 g) and PDMS (10.0 g) was quickly added to MDI using an addition funnel while stirring. After the addition was complete the reaction mixture was heated up to 80 °C and allowed to react for 2 hours with continuous stirring under nitrogen.

Prepared pre-polymer was first chain extended with 2.4154g of BHTD by slowly adding to the pre-polymer using a syringe and allowed to react for 2 hours while stirring under nitrogen. The reaction mixture was cooled down to 0 °C and 178 mL of anhydrous DMAc was added to get a clear solution. 5.212 × 10⁻¹ g of EDA in 3 mL of DMAc was added dropwise using a syringe into the cooled BHTD chain extended reaction mixture with slow stirring. After complete addition of EDA, the mixture was further reacted for 30 minutes to complete the reaction. When the solution viscosity increased, the stirring was stopped and the reaction mixture was heated up to 80 °C, the system was then stirred until the solution turned clear. Prepared PUU was transferred to a screw-capped glass bottle under nitrogen and stored at room temperature.

A similar procedure was followed to synthesise PUUs with all other linked macrodiols.

**Table 9** shows the material of the PUUs prepared along with sample abbreviation.

**Table 9 - Sample abbreviation and quantities of reagents used in preparing each of the polyurethaneureas**

| **Sample Abbreviation** | **PDMS or Linked PDMS^{a}** | **PHMO or Linked PHMO^{b}** | **MDI** | **BHTD** | **EDA** |
|---|---|---|---|---|---|
| PU-C | PDMS | PHMO | 7.49 | 2.25 | 0.4863 |
| PU-1 | PDMS | PHMO-MDI-PHMO | 7.29 | 2.41 | 0.5212 |
| PU-2 | PDMS-MDI-PDMS | PHMO | 6.97 | 2.68 | 0.5775 |
| PU-3 | PDMS | PHMO-HDI-PHMO | 7.30 | 2.41 | 0.5199 |
| PU-4 | PDMS-HDI-PDMS | PHMO | 6.94 | 2.70 | 0.5825 |
| PU-5 | PDMS | PHMO-IPDI-PHMO | 7.29 | 2.42 | 0.5212 |
| PU-6 | PDMS-IPDI-PDMS | PHMO | 6.93 | 2.71 | 0.5854 |

| | | | | | |
|---|---|---|---|---|---|
| ^{a}Amount used was 10.00 g; ^{b}amount used was 2.5 g | | | | | |

The molecular weight of synthesised polyurethanesureas was determined by gel permeation chromatography using procedures described under the heading "Equipment" and the results are summarized in **Table 10**.

**Table 10 - GPC molecular weights of synthesised poly(urethane-urea)**

| **Sample code** | ***Mₙ*** | ***M_{w}*** | ***M_{w}* / *Mₙ* (PDI)** |
|---|---|---|---|
| PU-C | 100813 | 328694 | 3.26 |
| PU-1 | 124147 | 314206 | 2.53 |
| PU-2 | 91048 | 245414 | 2.69 |
| PU-3 | 90471 | 260001 | 2.87 |
| PU-4 | 99649 | 355041 | 3.56 |
| PU-5 | 113923 | 347978 | 3.05 |
| PU-6 | 109531 | 262741 | 2.39 |

### Example 23 - Tensile testing of materials prepared in Example 22

Tensile testing was carried out using dumbbells punched from dried polyurethaneurea films cast from solutions with materials described in Example 22 (**Table 9**). Tensile testing was carried out on an Instron Universal Testing Machine. The results are summarised in **Table 11**.

The results indicate that the polyurethaneureas prepared from PHMO linked with MDI and IPDI were significantly high in tensile and tear strength compared to that prepared from unlinked PHMO.

**Table 11 - Tensile properties of polyurethaneurea series**

| **Polyurethane** | **Elongation at break (%)** | **Ultimate tensile stress (MPa)** | **Young's modulus (MPa)** | **Tear strength (N/mm)** |
|---|---|---|---|---|
| PU-C | 688 ± 47 | 16 ± 0.9 | 8 ± 0.7 | 32 ± 1.5 |
| PU-1 | 646 ± 24 | 31 ± 2.4 | 18 ± 0.7 | 64 ± 2.3 |
| PU-2 | 607 ± 6 | 16 ± 0.5 | 10 ± 0.3 | 65 ± 4.0 |
| PU-3 | 527 ± 51 | 12 ± 1.3 | 11 ± 0.7 | 38 ± 4.0 |
| PU-4 | 582 ± 35 | 15 ± 0.7 | 13 ± 0.6 | 52 ± 8.3 |
| PU-5 | 681 ± 47 | 35 ± 3.0 | 20 ± 1.3 | 61 ± 11.5 |
| PU-6 | 453 ± 38 | 13 ± 1.1 | 14 ± 0.6 | 37 ± 2.7 |

## Claims

1. A copolymer segment which is a block copolymer macro-monomer of Formula 1 for a thermoplastic polyurethane or polyurethaneurea elastomer material: 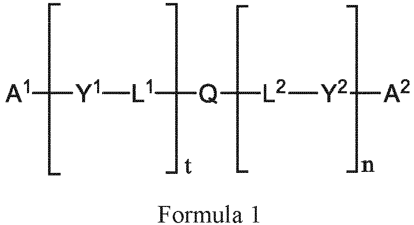 wherein
A¹ is an endcapping group, preferably the endcapping group is selected from the group consisting of hydroxyl and amine groups;
A² is hydrogen or an endcapping group, preferably the endcapping group is selected from the group consisting of hydroxyl and amine groups;
each Y¹ and Y² are independently selected from:
a polysiloxane macrodiol, preferably a polydimethylsiloxane (PDMS) diol;
polysiloxane macrodiamine;
polyether macrodiol, preferably selected from the group consisting of: poly(hexamethylene oxide) (PHMO), poly(heptamethylene oxide), poly(octamethylene oxide) (POMO) and poly(decamethylene oxide) (PDMO);
polycarbonate macrodiol;
polyester macrodiol; and
a polyhydrocarbon macrodiol, preferably selected from the group consisting of: poly(isobutylene)diol, poly(butadiene)diol and hydrogenated poly(butadiene diol);
each L¹ and L² is a divalent linking group independently selected from urethane, urea and phosphonate;
n is an integer of 1 to 5;
t is an integer of 0 to 5, preferably t = 0; and
Q is selected from a moiety of Formula A or Formula B: 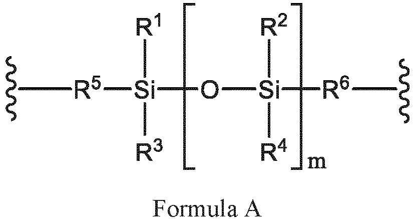 wherein
R¹, R², R³, and R⁴, are each independently selected from hydrogen and an optionally substituted straight chain, branched or cyclic, saturated and unsaturated hydrocarbon radical; preferably R¹, R², R³, and R⁴, are each methyl;
R⁵ and R⁶ are each independently selected from a straight chain, branched or cyclic, saturated and unsaturated hydrocarbon radical optionally interrupted with one or more heteroatoms independently selected from O, N and S;
m is an integer of 2 to 50; or 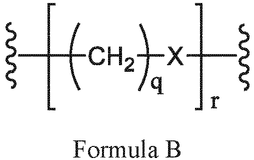 wherein
X is a group selected from OC(O)O, C(O)O and O;
q is an integer of 1 to 50; and
r is an integer of 2 to 50,
wherein optionally substituted means optionally substituted with one or more substituents independently selected from: oxygen, nitrogen, sulphur, alkyl, alkenyl, alkynyl, aryl, halo, haloalkyl, haloalkenyl, haloalkynyl, haloaryl, hydroxy, alkoxy, alkenyloxy, alkynyloxy, aryloxy, carboxy, benzyloxy, haloalkoxy, haloalkenyloxy, haloalkynyloxy, haloaryloxy, nitro, nitroalkyl, nitroalkenyl, nitroalkynyl, nitroaryl, nitroheterocyclyl, azido, amino, alkylamino, alkenylamino, alkynylamino, arylamino, benzylamino, acyl, alkenylacyl, alkynylacyl, arylacyl, acylamino, acyloxy, aldehydo, alkylsulphonyl, arylsulphonyl, alkylsulphonylamino, arylsulphonylamino, alkylsulphonyloxy, arylsulphonyloxy, heterocyclyl, heterocycloxy, heterocyclylamino, haloheterocyclyl, alkylsulphenyl, arylsulphenyl, carboalkoxy, carboaryloxy, mercapto, alkylthio, arylthio, and acylthio groups.

2. The copolymer segment of claim 1, wherein Y¹ and Y² are each independently selected from a moiety of Formula A' or Formula B': 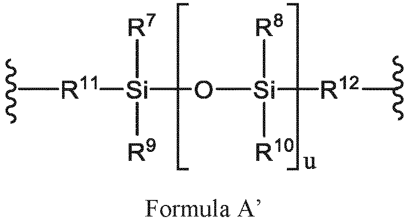 wherein
R⁷, R⁸, R⁹, and R¹⁰, are each independently selected from hydrogen and an optionally substituted straight chain, branched or cyclic, saturated and unsaturated hydrocarbon radical; preferably R⁷, R⁸, R⁹, and R¹⁰, are each methyl;
R¹¹ and R¹² are each independently selected from a straight chain, branched or cyclic, saturated and unsaturated hydrocarbon radical optionally interrupted with one or more heteroatoms independently selected from O, N and S; and
u is an integer of 1 to 50; or 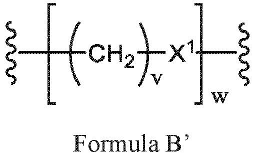 wherein
X¹ is a group selected from OC(O)O, C(O)O and O;
v is an integer of 1 to 50, for example an integer of 5 to 20; and
w is an integer of 2 to 50.

3. The copolymer segment of claim 1 or claim 2, wherein L¹ and L² are the same.

4. The copolymer segment of any one of claims 1 to 3, wherein Y¹ and Y² are the same.

5. The copolymer segment of any one of claims 1 to 4, wherein at least one of R⁵ and R⁶ is independently selected from the moiety of Formula D': 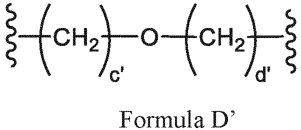 wherein c' is an integer between 1 and 6; and d' is an integer between 1 and 6.

6. The copolymer segment of claim 1, wherein the copolymer segment is a block copolymer segment of Formula 1A: 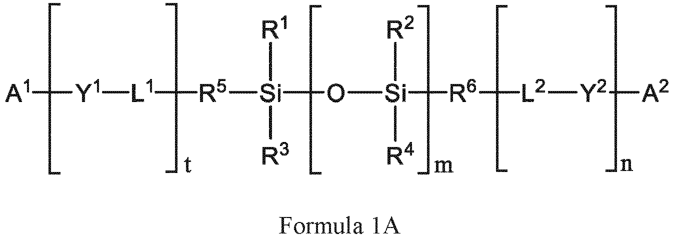 wherein each of A¹, A², Y¹, L¹, L², Y², R¹, R², R³, R⁴, R⁵, R⁶, m, n and t, are as defined in claim 1.

7. The copolymer segment of claim 1, wherein the copolymer segment is a block copolymer segment of Formula 1A(ii): 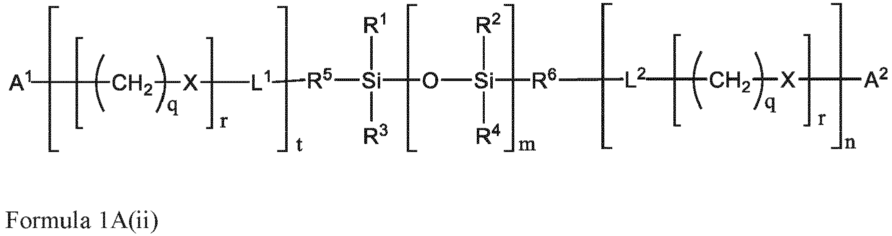 wherein each of A¹, A², L¹, L², R¹, R², R³, R⁴, R⁵, R⁶, X, m, q, r and t, are as defined in claim 1.

8. The copolymer segment of claim 1, wherein the copolymer segment is a block copolymer segment of Formula 1B: 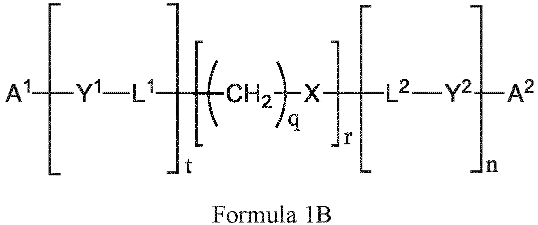 wherein each of A¹, A², Y¹, L¹, L², Y², X, n, r, t, and q, are as defined in claim 1.

9. The copolymer segment of claim 1, wherein the copolymer segment is a block copolymer segment of Formula 1B(ii): 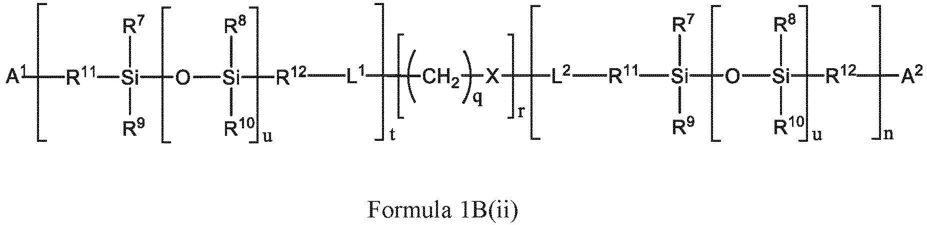
wherein each of A¹, A², L¹, L², X, n, q, r and t, are as defined in claim 1, and
R⁷, R⁸, R⁹, and R¹⁰, are each independently selected from hydrogen and an optionally substituted straight chain, branched or cyclic, saturated and unsaturated hydrocarbon radical; preferably R⁷, R⁸, R⁹, and R¹⁰, are each methyl;
R¹¹ and R¹² are each independently selected from a straight chain, branched or cyclic, saturated and unsaturated hydrocarbon radical optionally interrupted with one or more heteroatoms independently selected from O, N and S; and
u is an integer of 1 to 50.

10. A thermoplastic polyurethane or polyurethaneurea elastomer material comprising a plurality of soft segments and hard segments, wherein the plurality of soft segments are each derived from a macro-monomer having at least one copolymer segment of Formula 1 according to claim 1 and optionally an additional polyol or polyamine, with the proviso that the polyurethane or polyurethaneurea elastomer material is not crosslinked.

11. The thermoplastic polyurethane or polyurethaneurea elastomer material of claim 10, wherein the thermoplastic polyurethane or polyurethaneurea elastomer material comprises a reaction product of:
i. at least one copolymer segment of Formula 1 according to any one of claim 1;
ii. a diisocyanate, preferably the diisocyanate is selected from the group consisting of: 4,4'-methylenediphenyl diisocyanate (MDI), methylene bis (cyclohexyl) diisocyanate (H12MDI), p-phenylene diisocyanate (p-PDI), trans-cyclohexane-1,4-diisocyanate (CHDI) or a mixture of the cis and trans isomers, 1,6-hexamethylene diisocyanate (HDI), 2,4-toluene diisocyanate (2,4-TDI), p-tetramethylxylene diisocyanate (p-TMXDI), isophorone diisocyanate and m-tetramethylxylene diisocyanate (m-TMXDI);
iii. one or more chain extenders, preferably the at least one chain extender is selected from the group consisting of: 1,4-butanediol, 1,6-hexanediol, 1,8-octanediol, 1,9-nonanediol, 1,10-decanediol 1,4-cyclohexane dimethanol, p-xyleneglycol, 1,4-bis (2-hydroxyethoxy) benzene, 1,12-dodecanediol, 1,3 bis-(4-hydroxybutyl) 1,1,3,3-tetramethyldisiloxane, ethylene diamine, ethanolamine, butanediamine, propanediamine, 1,3 bis-(3-aminopropyl) tetramethyldisiloxane, and 1,3 bis-(3-aminobutyl) tetramethyldisiloxane; and
iv. optionally an additional polyol or polyamine,
with the proviso that the polyurethane or polyurethaneurea elastomer material is not crosslinked.

12. An article which is composed wholly or partly of the polyurethane or polyurethaneurea elastomer material of claim 10 or claim 11, preferably the article is selected from the group consisting of: artificial leather, shoe soles, cable sheathing, varnishes, coatings, structural components for pumps or vehicles, mining ore screens, conveyor belts, laminating compounds, fibres, textiles, separation membranes, sealants, adhesive components, and a medical device, preferably the medical device is selected from the group consisting of: an implant, a cardiac pacemaker, defibrillator, catheter, heart valve, cardiac assist device, vascular graft, an implantable prosthesis, a cannula, extra-corporeal device, artificial organ, pacemaker lead, defibrillator lead, blood pump, balloon pump, A-V shunt, biosensor, membrane for cell encapsulation, drug delivery device, wound dressing, artificial joint, orthopaedic implant, and a soft tissue replacement.

13. The article according to claim 12, wherein the article is a heart valve, the valve comprising:
• a plurality of valve leaflets; and
• a support structure,
wherein one or more of the valve leaflets are composed wholly or partly of the polyurethane or polyurethane urea elastomer material of claim 10 or claim 11.

14. A process for preparing a thermoplastic polyurethane or polyurethaneurea elastomer material comprising the copolymer segment of Formula 1 according to any one of claims 1 to 9, wherein the process comprises the steps of:
i. providing a copolymer segment of Formula 1 according to claim 1;
ii. optionally reacting the material of step i. with a divalent compound and: at least one macrodiol; at least one macrodiamine; or a mixture of at least one macrodiol and at least one macrodiamine; and
iii. reacting the copolymer segment of step i. or step ii. with a chain extender or a mixture of chain extenders to form the polyurethane or polyurethaneurea elastomer.

15. The copolymer segment of claim 1, wherein A¹ is a hydroxyl or an amine group; A² is hydrogen, a hydroxyl, or an amine group, and wherein when t = 0 and n = 1, Q and Y² are the same.

16. The copolymer segment of claim 1, wherein A¹ is a hydroxyl or an amine group; A² is hydrogen, a hydroxyl, or an amine group; and q is an integer of 3 to 50, wherein when t = 0 and n = 1, Q and Y² are the same.

## Patentansprüche

1. Copolymer-Segment, das ein Block-Copolymer-Makromonomer der Formel 1 für ein thermoplastisches Polyurethan- oder Polyurethanharnstoff-Elastomermaterial ist: 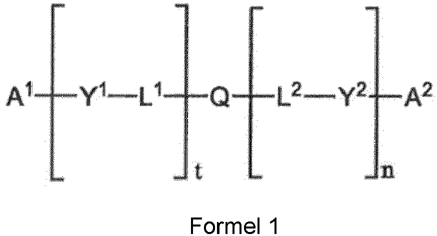 worin:
A¹ eine Endverkappungsgruppe ist, wobei die Endverkappungsgruppe vorzugsweise aus der aus Hydroxyl- und Aminogruppen bestehenden Gruppe ausgewählt ist;
A² Wasserstoff oder eine Endverkappungsgruppe ist, wobei die Endverkappungsgruppe vorzugsweise aus der aus Hydroxyl- und Aminogruppen bestehenden Gruppe ausgewählt ist;
Y¹ und Y² jeweils unabhängig aus Folgendem ausgewählt sind:
einem Polysiloxanmakrodiol, vorzugsweise einem Polydimethylsiloxan- (PDMS-) Diol;
Polysiloxanmakrodiamin;
Polyethermakrodiol, vorzugsweise ausgewählt aus der aus Poly(hexamethylenoxid) (PHMO), Poly(heptamethylenoxid), Poly(octamethylenoxid) (POMO) und Poly(decamethylenoxid) (PDMO) bestehenden Gruppe;
Polycarbonatmakrodiol;
Polyestermakrodiol; und
einem Polykohlenwasserstoffmakrodiol, vorzugsweise ausgewählt aus der aus den folgenden bestehenden Gruppe: Poly(isobutylen)diol, Poly(butadien)diol und hydriertem Poly(butadiendiol);
L¹ und L² jeweils eine zweiwertige Linkergruppe sind, die jeweils unabhängig aus Urethan, Harnstoff und Phosphonat ausgewählt ist;
n eine ganze Zahl von 1 bis 5 ist;
t eine ganze Zahl von 0 bis 5 ist, wobei vorzugsweise t = 0 ist; und
Q aus einer Gruppierung der Formel A oder Formel B ausgewählt ist: 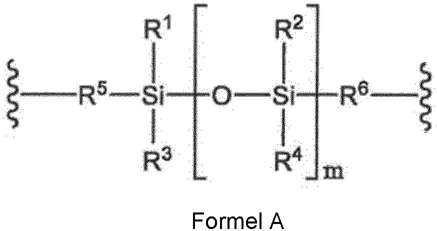 worin:
R¹, R², R³ und R⁴ jeweils unabhängig aus Wasserstoff und einem gegebenenfalls substituierten unverzweigten, verzweigten oder zyklischen, gesättigten oder ungesättigten Kohlenwasserstoffrest ausgewählt sind; wobei R¹, R², R³ und R⁴ jeweils vorzugsweise Methyl sind;
R⁵ und R⁶ jeweils unabhängig aus einem unverzweigten, verzweigten oder zyklischen, gesättigten oder ungesättigten Kohlenwasserstoffrest ausgewählt sind, der gegebenenfalls durch ein oder mehrere Heteroatome unterbrochen ist, die jeweils unabhängig aus O, N und S ausgewählt sind; und
m eine ganze Zahl von 2 bis 50 ist; oder 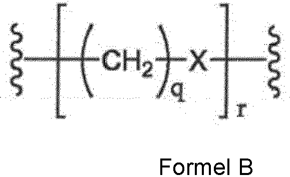 worin:
X eine aus OC(O)O, C(O)O und O ausgewählte Gruppe ist;
q eine ganze Zahl von 1 bis 50 ist; und
r eine ganze Zahl von 2 bis 50 ist,
wobei gegebenenfalls substituiert gegebenenfalls mit einem oder mehreren Substituenten substituiert bedeutet, die jeweils unabhängig aus den folgenden ausgewählt sind: Sauerstoff-, Stickstoff-, Schwefel-, Alkyl-, Alkenyl-, Alkinyl-, Aryl-, Halogen-, Halogenalkyl-, Halogenalkenyl-, Halogenalkinyl-, Halogenaryl-, Hydroxy-, Alkoxy-, Alkenyloxy-, Alkinyloxy-, Aryloxy-, Carboxy-, Benzyloxy-, Halogenalkoxy-, Halogenalkenyloxy-, Halogenalkinyloxy-, Halogenaryloxy-, Nitro-, Nitroalkyl-, Nitroalkenyl-, Nitroalkinyl-, Nitroaryl-, Nitroheterocyclyl-, Azido-, Amino-, Alkylamino-, Alkenylamino-, Alkinylamino-, Arylamino-, Benzylamino-, Acyl-, Alkenylacyl-, Alkinylacyl-, Arylacyl-, Acylamino-, Acyloxy-, Aldehydo-, Alkylsulfonyl-, Arylsulfonyl-, Alkylsulfonylamino-, Arylsulfonylamino-, Alkylsulfonyloxy-, Arylsulfonyloxy-, Heterocyclyl-, Heterocycloxy-, Heterocyclylamino-, Halogenheterocyclyl-, Alkylsulfenyl-, Arylsulfenyl-, Carboalkoxy-, Carboaryloxy-, Mercapto-, Alkylthio-, Arylthio- und Acylthiogruppen.

2. Copolymer-Segment nach Anspruch 1, worin Y¹ und Y² jeweils unabhängig aus einer Gruppierung der Formel A' oder Formel B' ausgewählt sind: 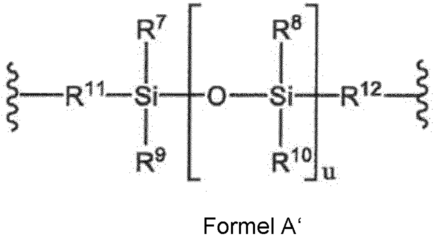 worin:
R⁷, R⁸, R⁹ und R¹⁰ jeweils unabhängig aus Wasserstoff und einem gegebenenfalls substituierten, unverzweigten, verzweigten oder zyklischen, gesättigten und ungesättigten Kohlenwasserstoffrest ausgewählt sind; wobei R⁷, R⁸, R⁹ und R¹⁰ vorzugsweise jeweils Methyl sind;
R¹¹ und R¹² jeweils unabhängig aus einem unverzweigten, verzweigten oder zyklischen, gesättigten und ungesättigten Kohlenwasserstoffrest ausgewählt sind, der gegebenenfalls durch ein oder mehrere Heteroatome unterbrochen ist, die jeweils unabhängig aus O, N und S ausgewählt sind; und
u eine ganze Zahl von 1 bis 50 ist; oder 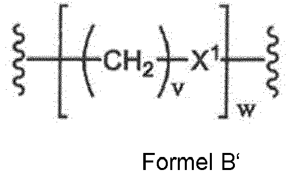 worin:
X¹ eine aus OC(O)O, C(O)O und O ausgewählte Gruppe ist;
v eine ganze Zahl von 1 bis 50 ist, z.B. eine ganze Zahl von 5 bis 20; und
w eine ganze Zahl von 2 bis 50 ist.

3. Copolymer-Segment nach Anspruch 1 oder Anspruch 2, worin L¹ und L² gleich sind.

4. Copolymer-Segment nach einem der Ansprüche 1 bis 3, worin Y¹ und Y² gleich sind.

5. Copolymer-Segment nach einem der Ansprüche 1 bis 4, wobei zumindest einer von R⁵ und R⁶ jeweils unabhängig aus einer Gruppierung der Formel D ausgewählt ist: 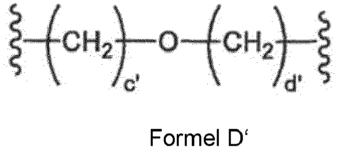 worin c' eine ganze Zahl zwischen 1 und 6 ist; und d' eine ganze Zahl zwischen 1 und 6 ist.

6. Copolymer-Segment nach Anspruch 1, wobei das Copolymer-Segment ein Block-Copolymer-Segment der Formel 1A ist: 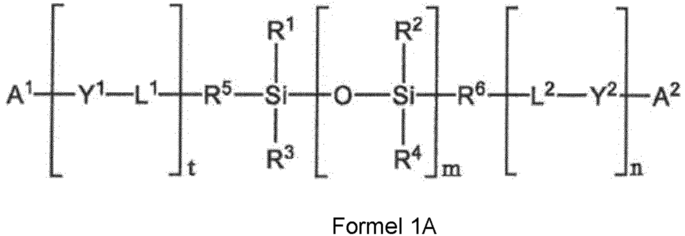 worin A¹, A², Y¹, L¹, L², Y², R¹, R², R³, R⁴, R⁵, R⁶, m, n und t jeweils wie in Anspruch 1 definiert sind.

7. Copolymer-Segment nach Anspruch 1, wobei das Copolymer-Segment ein Block-Copolymer-Segment der Formel 1A(ii) ist: 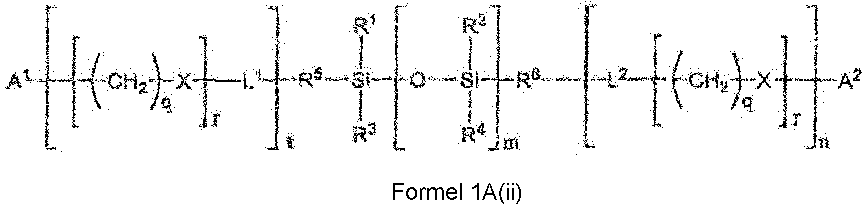 worin A¹, A², L¹, L², R¹, R², R³, R⁴, R⁵, R⁶, X, m, q, r und t jeweils wie in Anspruch 1 definiert sind.

8. Copolymer-Segment nach Anspruch 1, wobei das Copolymer-Segment ein Block-Copolymer-Segment der Formel 1 B ist: 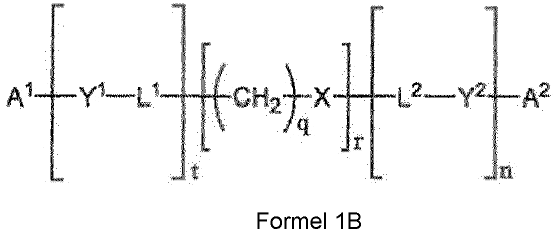 worin A¹, A², Y¹, L¹, L², Y², X, n, r, t und q jeweils wie in Anspruch 1 definiert sind.

9. Copolymer-Segment nach Anspruch 1, wobei das Copolymer-Segment ein Block-Copolymer-Segment der Formel 1B(ii) ist: 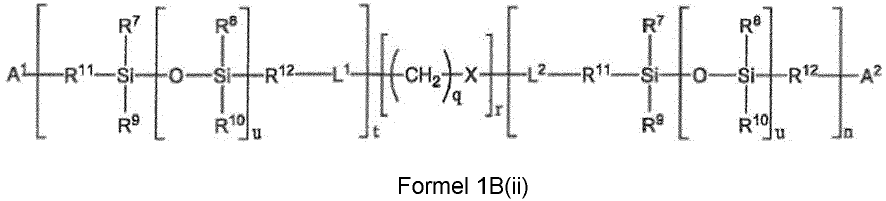
worin A¹, A², L¹, L², X, n, q, r und t jeweils wie in Anspruch 1 definiert sind und
R⁷, R⁸, R⁹ und R¹⁰ jeweils unabhängig aus Wasserstoff und einem gegebenenfalls substituierten, unverzweigten, verzweigten oder zyklischen, gesättigten oder ungesättigten Kohlenwasserstoffrest ausgewählt sind; wobei R⁷, R⁸, R⁹ und R¹⁰ jeweils vorzugsweise Methyl sind;
R¹¹ und R¹² jeweils unabhängig aus einem unverzweigten, verzweigten oder zyklischen, gesättigten oder ungesättigten Kohlenwasserstoffrest ausgewählt sind, der gegebenenfalls durch ein oder mehrere Heteroatome unterbrochen ist, die jeweils unabhängig aus O, N und S ausgewählt sind; und
u eine ganze Zahl von 1 bis 50 ist.

10. Thermoplastisches Polyurethan- oder Polyurethanharnstoff-Elastomermaterial, das eine Vielzahl von weichen Segmenten und harten Segmenten umfasst, wobei die Vielzahl von weichen Segmenten jeweils von einem Makromonomer mit zumindest einem Copolymer-Segment der Formel 1 nach Anspruch 1 und gegebenenfalls einem zusätzlichen Polyol oder Polyamin abgeleitet ist,
mit der Maßgabe, dass das Polyurethan- oder Polyurethanharnstoff-Elastomermaterial nicht vernetzt ist.

11. Thermoplastisches Polyurethan- oder Polyurethanharnstoff-Elastomermaterial nach Anspruch 10, wobei das thermoplastische Polyurethan- oder Polyurethanharnstoff-Elastomermaterial ein Reaktionsprodukt von Folgendem umfasst:
i) zumindest einem Copolymer-Segment der Formel 1 nach Anspruch 1;
ii) einem Diisocyanat, wobei das Diisocyanat vorzugsweise aus der aus den folgenden bestehenden Gruppe ausgewählt ist: 4,4'-Methylendiphenyldiisocyanat (MDI), Methylen-bis(cyclohexyl)diisocyanat (H12MDI), p-Phenylendiisocyanat (p-PDI), trans-Cyclohexan-1,4-diisocyanat (CHDI) oder einem Gemisch der cis- und trans-Isomere, 1,6-Hexamethylendiisocyanat (HDI), 2,4-Toluoldiisocyanat (2,4-TDI), p-Tetramethylxyloldiisocyanat (p-TMXDI), Isophorondiisocyanat und m-Tetramethylxylendiisocyanat (m-TMXDI);
iii) einem oder mehreren Kettenverlängerern, wobei der zumindest eine Kettenverlängerer vorzugsweise aus der aus den folgenden bestehenden Gruppe ausgewählt ist: 1,4-Butandiol, 1,6- Hexandiol, 1,8-Octandiol, 1,9-Nonandiol, 1,10-Decandiol 1,4-Cyclohexandimethanol, p-Xylolglykol, 1,4-Bis(2-hydroxyethoxy)benzol, 1,12-Dodecandiol, 1,3-Bis-(4-hydroxybutyl)-1,1,3,3-tetramethyldisiloxan, Ethylendiamin, Ethanolamin, Butandiamin, Propandiamin, 1,3-Bis(3-aminopropyl)tetramethyldisiloxan und 1,3-Bis(3-aminobutyl)tetramethyldisiloxan; und
iv) gegebenenfalls einem zusätzlichen Polyol oder Polyamin,
mit der Maßgabe, dass das Polyurethan- oder Polyurethanharnstoff-Elastomermaterial nicht vernetzt ist.

12. Gegenstand, der vollständig oder teilweise aus einem Polyurethan- oder Polyurethanharnstoff-Elastomermaterial nach Anspruch 10 oder 11 besteht, wobei der Gegenstand vorzugsweise aus der aus den folgenden bestehenden Gruppe ausgewählt ist: künstlichem Leder, Schuhsohlen, Kabelhüllen, Lacken, Beschichtungen, Strukturkomponenten für Pumpen oder Fahrzeuge, Erzabbausieben, Förderbändern, Laminierverbindungen, Fasern, Textilien, Trennmembranen, Dichtungsmitteln, Klebstoffkomponenten und medizinischen Vorrichtungen, wobei die medizinischen Vorrichtungen vorzugsweise aus der aus den folgenden bestehenden Gruppe ausgewählt sind: einem Implantat, einem Herzschrittmacher, einem Defibrillator, einem Katheter, einer Herzklappe, einem Herzunterstützungsgerät, einem Gefäßtransplantat, einer implantierbaren Prothese, einer Kanüle, einer extrakorporalen Vorrichtung, einem künstlichen Organ, einem Herzschrittmacherdraht, einem Defibrillatordraht, einer Blutpumpe, einer Ballonpumpe, einem A-V-Shunt, einem Biosensor, einer Membran zur Zelleinkapselung, einer Arzneimittelzufuhrvorrichtung, einem Wundverband, einem künstlichen Gelenk, einem orthopädischen Implantat und einem Weichteilersatz.

13. Gegenstand nach Anspruch 12, wobei der Gegenstand eine Herzklappe ist, wobei die Herzklappe Folgendes umfasst:
• eine Vielzahl von Klappensegeln; und
• eine Trägerstruktur,
wobei eines oder mehrere der Klappensegel vollständig oder teilweise aus einem Polyurethan- oder Polyurethanharnstoff-Elastomermaterial nach Anspruch 10 oder 11 bestehen.

14. Verfahren zur Herstellung eines thermoplastischen Polyurethan- oder Polyurethanharnstoff-Elastomermaterials, das ein Copolymer-Segment der Formel 1 nach einem der Ansprüche 1 bis 9 umfasst, wobei das Verfahren die folgenden Schritte umfasst:
i) das Bereitstellen eines Copolymer-Segments der Formel 1 nach Anspruch 1;
ii) gegebenenfalls das Umsetzen des Materials aus Schritt i) mit einer zweiwertigen Verbindung und zumindest einem Makrodiol, zumindest einem Makrodiamin oder einem Gemisch aus zumindest einem Makrodiol und zumindest einem Makrodiamin; und
iii) das Umsetzen des Copolymer-Segments aus Schritt i) oder Schritt ii) mit einem Kettenverlängerer oder einem Gemisch aus Kettenverlängerern, um das Polyurethan- oder Polyurethanharnstoff-Elastomer zu bilden.

15. Copolymer-Segment nach Anspruch 1, worin A¹ eine Hydroxyl- oder Aminogruppe ist; A² Wasserstoff, eine Hydroxyl- oder eine Aminogruppe ist; und worin, wenn t = 0 ist und n = 1 ist, Q und Y² gleich sind.

16. Copolymer-Segment nach Anspruch 1, worin A¹ eine Hydroxyl- oder Aminogruppe ist; A² Wasserstoff, eine Hydroxyl- oder eine Aminogruppe ist; und q eine ganze Zahl von 3 bis 50 ist; wobei, wenn t = 0 ist und n = 1 ist, Q und Y² gleich sind.

## Revendications

1. Segment de copolymère qui est un macromonomère de copolymère séquencé de formule 1 pour un matériau élastomère de polyuréthane ou polyuréthane-urée thermoplastique : 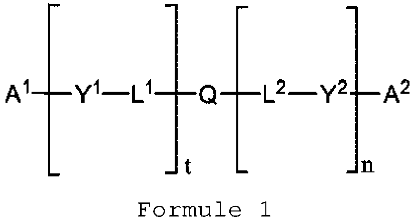 dans laquelle
A¹ est un groupe de coiffe d'extrémité, de préférence le groupe de coiffe d'extrémité est choisi dans le groupe constitué de groupes hydroxyle et aminé ;
A² est hydrogène ou un groupe de coiffe d'extrémité, de préférence le groupe de coiffe d'extrémité est choisi dans le groupe constitué de groupes hydroxyle et aminé ;
chaque Y¹ et Y² est indépendamment choisi parmi :
un polysiloxane macrodiol, de préférence un polydiméthylsiloxane (PDMS) diol ;
une polysiloxane macrodiamine ;
un polyéther macrodiol, de préférence choisi dans le groupe constitué de : poly(oxyde d'hexaméthylène) (PHMO), poly(oxyde d'heptaméthylène), poly(oxyde d'octaméthylène) (POMO) et poly(oxyde de décaméthylène) (PDMO) ;
un polycarbonate macrodiol ;
un polyester macrodiol ; et
un polyhydrocarbure macrodiol, de préférence choisi dans le groupe constitué de : poly(isobutylène)diol, poly(butadiène)diol et poly(butadiène diol) hydrogéné ;
chaque L¹ et L² est un groupe de liaison divalent indépendamment choisi parmi uréthane, urée et phosphonate ;
n est un entier de 1 à 5 ;
t est un entier de 0 à 5, de préférence t = 0 ; et
Q est choisi parmi un fragment de formule A ou formule B : 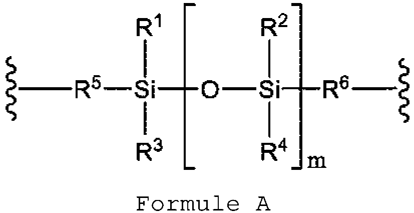 dans laquelle
R¹, R², R³ et R⁴, sont chacun indépendamment choisis parmi hydrogène et un radical hydrocarboné saturé et insaturé à chaîne linéaire, ramifié ou cyclique facultativement substitué ; de préférence R¹, R², R³ et R⁴ sont chacun méthyle ;
R⁵ et R⁶ sont chacun indépendamment choisis parmi un radical hydrocarboné saturé et insaturé à chaîne linéaire, ramifié ou cyclique facultativement interrompu par un ou plusieurs hétéroatomes indépendamment choisis parmi O, N et S ;
m est un entier de 2 à 50 ; ou 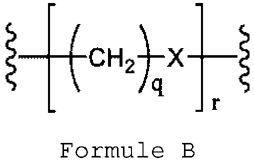 dans laquelle
X est un groupe choisi parmi OC(O)O, C(O)O et O ;
q est un entier de 1 à 50 ; et
r est un entier de 2 à 50,
où facultativement substitué signifie facultativement substitué par un ou plusieurs substituants indépendamment choisis parmi : des groupes oxygène, azote, soufre, alkyle, alcényle, alcynyle, aryle, halogéno, halogénoalkyle, halogénoalcényle, halogénoalcynyle, halogénoaryle, hydroxy, alcoxy, alcényloxy, alcynyloxy, aryloxy, carboxy, benzyloxy, halogénoalcoxy, halogénoalcényloxy, halogénoalcynyloxy, halogénoaryloxy, nitro, nitroalkyle, nitroalcényle, nitroalcynyle, nitroaryle, nitrohétérocyclyle, azido, amino, alkylamino, alcénylamino, alcynylamino, arylamino, benzylamino, acyle, alcénylacyle, alcynylacyle, arylacyle, acylamino, acyloxy, aldéhydo, alkylsulfonyle, arylsulfonyle, alkylsulfonylamino, arylsulfonylamino, alkylsulfonyloxy, arylsulfonyloxy, hétérocyclyle, hétérocycloxy, hétérocyclylamino, halohétérocyclyle, alkylsulfényle, arylsulfényle, carboalcoxy, carboaryloxy, mercapto, alkylthio, arylthio et acylthio.

2. Segment de copolymère selon la revendication 1, dans lequel Y¹ et Y² sont chacun indépendamment choisis parmi un fragment de formule A' ou formule B' : 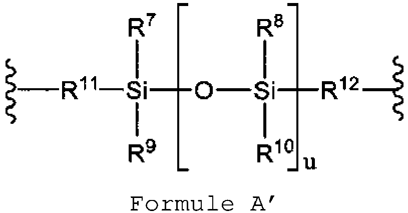 dans laquelle
R⁷, R⁸, R⁹ et R¹⁰, sont chacun indépendamment choisis parmi hydrogène et un radical hydrocarboné saturé et insaturé à chaîne linéaire, ramifié ou cyclique facultativement substitué ; de préférence R⁷, R⁸, R⁹ et R¹⁰, sont chacun méthyle ;
R¹¹ et R¹² sont chacun indépendamment choisis parmi un radical hydrocarboné saturé et insaturé à chaîne linéaire, ramifié ou cyclique facultativement interrompu par un ou plusieurs hétéroatomes indépendamment choisis parmi O, N et S ; et
u est un entier de 1 à 50 ; ou 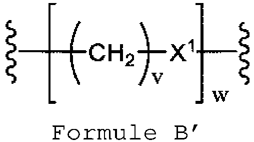 dans laquelle
X¹ est un groupe choisi parmi OC(O)O, C(O)O et O ;
v est un entier de 1 à 50, par exemple un entier de 5 à 20 ; et
w est un entier de 2 à 50.

3. Segment de copolymère selon la revendication 1 ou la revendication 2, dans lequel L¹ et L² sont identiques.

4. Segment de copolymère selon l'une quelconque des revendications 1 à 3, dans lequel Y¹ et Y² sont identiques.

5. Segment de copolymère selon l'une quelconque des revendications 1 à 4, dans lequel au moins l'un de R⁵ et R⁶ est indépendamment choisi parmi le fragment de formule D' : 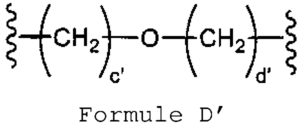 dans laquelle c' est un entier compris entre 1 et 6 ; et d' est un entier compris entre 1 et 6.

6. Segment de copolymère selon la revendication 1, où le segment de copolymère est un segment de copolymère séquencé de formule 1A : 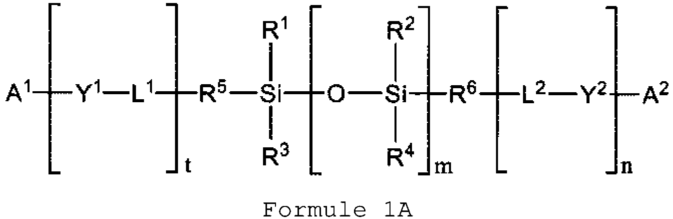 dans laquelle chacun de A¹, A², Y¹, L¹, L², Y², R¹, R², R³, R⁴, R⁵, R⁶, m, n et t est tel que défini dans la revendication 1.

7. Segment de copolymère selon la revendication 1, où le segment de copolymère est un segment de copolymère séquencé de formule 1A(ii) : 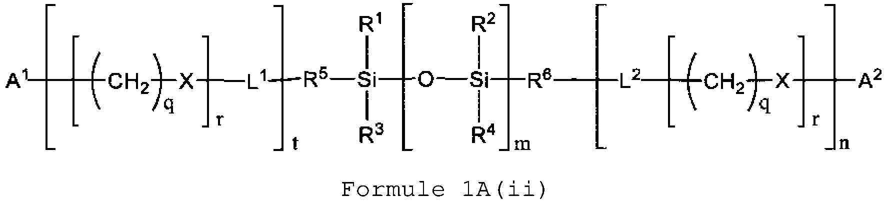 dans laquelle chacun de A¹, A², L¹, L², R¹, R², R³, R⁴, R⁵, R⁶, X, m, q, r et t est tel que défini dans la revendication 1.

8. Segment de copolymère selon la revendication 1, où le segment de copolymère est un segment de copolymère séquencé de formule 1B : 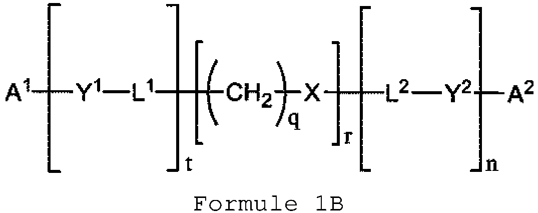 dans laquelle chacun de A¹, A², Y¹, L¹, L², Y², X, n, r, t, et q est tel que défini dans la revendication 1.

9. Segment de copolymère selon la revendication 1, où le segment de copolymère est un segment de copolymère séquencé de formule 1B(ii) : 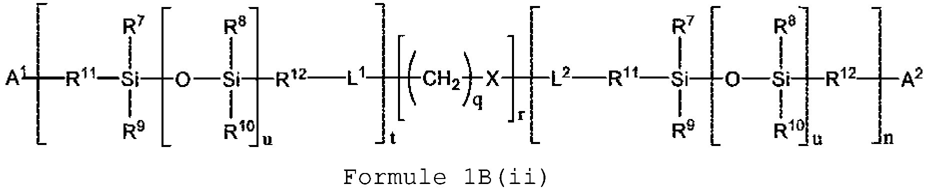
dans laquelle chacun de A¹, A², L¹, L², X, n, q, r et t est tel que défini dans la revendication 1, et
R⁷, R⁸, R⁹ et R¹⁰, sont chacun indépendamment choisis parmi hydrogène et un radical hydrocarboné saturé et insaturé à chaîne linéaire, ramifié ou cyclique facultativement substitué ; de préférence R⁷, R⁸, R⁹ et R¹⁰ sont chacun méthyle ;
R¹¹ et R¹² sont chacun indépendamment choisis parmi un radical hydrocarboné saturé et insaturé à chaîne linéaire, ramifié ou cyclique facultativement interrompu par un ou plusieurs hétéroatomes indépendamment choisis parmi O, N et S ; et
u est un entier de 1 à 50.

10. Matériau élastomère de polyuréthane ou polyuréthane-urée thermoplastique comprenant une pluralité de segments souples et de segments rigides, dans lequel la pluralité de segments souples sont chacun dérivés d'un macromonomère ayant au moins un segment de copolymère de formule 1 selon la revendication 1 et facultativement un polyol ou une polyamine supplémentaire, à condition que le matériau élastomère de polyuréthane ou polyuréthane-urée ne soit pas réticulé.

11. Matériau élastomère de polyuréthane ou polyuréthane-urée thermoplastique selon la revendication 10, où le matériau élastomère de polyuréthane ou polyuréthane-urée thermoplastique comprend un produit de réaction de :
i. au moins un segment de copolymère de formule 1 selon la revendication 1 ;
ii. un diisocyanate, de préférence le diisocyanate est choisi dans le groupe constitué des : diisocyanate de 4,4'-méthylènediphényle (MDI), diisocyanate de méthylène-bis (cyclohexyle) (H12MDI), diisocyanate de p-phénylène (p-PDI), trans-1,4-diisocyanate de cyclohexane (CHDI) ou un mélange des isomères cis et trans, diisocyanate de 1, 6-hexaméthylène (HDI), diisocyanate de 2,4-toluène (2,4-TDI), diisocyanate de p-tétraméthylxylène (p-TMXDI), diisocyanate d'isophorone et diisocyanate de m-tétraméthylxylène (m-TMXDI) ;
iii. un ou plusieurs extenseurs de chaîne, de préférence l'au moins un extenseur de chaîne est choisi dans le groupe constitué des : 1,4-butanediol, 1,6-hexanediol, 1,8-octanediol, 1,9-nonanediol, 1,10-décanediol 1,4-cyclohexanediméthanol, p-xylèneglycol, 1,4-bis-(2-hydroxyéthoxy)benzène, 1,12-dodécanediol, 1,3-bis-(4-hydroxybutyl)-1,1,3,3-tétraméthyldisiloxane, éthylènediamine, éthanolamine, butanediamine, propanediamine, 1,3-bis-(3-aminopropyl)tétraméthyldisiloxane et 1,3-bis-(3-aminobutyl)tétraméthyldisiloxane ; et
iv. facultativement un polyol ou une polyamine supplémentaire,
à condition que le matériau élastomère de polyuréthane ou polyuréthane-urée ne soit pas réticulé.

12. Article qui est composé complètement ou partiellement du matériau élastomère de polyuréthane ou polyuréthane-urée selon la revendication 10 ou la revendication 11, de préférence l'article est choisi dans le groupe constitué de : cuir artificiel, semelles de chaussure, gaine de câble, vernis, revêtements, composants structurels pour pompes ou véhicules, tamis de minerai d'extraction, bandes transporteuses, composés de stratification, fibres, textiles, membranes de séparation, produits d'étanchéité, composants adhésifs et dispositif médical, de préférence le dispositif médical est choisi dans le groupe constitué de : un implant, un stimulateur cardiaque, un défibrillateur, un cathéter, une valve cardiaque, un dispositif d'assistance cardiaque, une greffe vasculaire, une prothèse implantable, une canule, un dispositif extracorporel, un organe artificiel, une dérivation de stimulateur, une dérivation de défibrillateur, une pompe à sang, une pompe à ballonnet, un shunt A-V, un biocapteur, une membrane pour encapsulation cellulaire, un dispositif d'administration de médicament, un pansement de plaie, une articulation artificielle, un implant orthopédique et un substitut de tissu mou.

13. Article selon la revendication 12, où l'article est une valve cardiaque, la valve comprenant :
• une pluralité de feuillets de valve ; et
• une structure de support,
dans lequel un ou plusieurs des feuillets de valve sont composés complètement ou partiellement du matériau élastomère de polyuréthane ou polyuréthane-urée selon la revendication 10 ou la revendication 11.

14. Procédé de préparation d'un matériau élastomère de polyuréthane ou polyuréthane-urée thermoplastique comprenant le segment de copolymère de formule 1 selon l'une quelconque des revendications 1 à 9, le procédé comprenant les étapes de :
i. fourniture d'un segment de copolymère de formule 1 selon la revendication 1 ;
ii. facultativement réaction du matériau de l'étape i. avec un composé divalent et : au moins un macrodiol ; au moins une macrodiamine ; ou un mélange d'au moins un macrodiol et d'au moins une macrodiamine ; et
iii. réaction du segment de copolymère de l'étape i. ou l'étape ii. avec un extenseur de chaîne ou un mélange d'extenseurs de chaîne pour former l'élastomère de polyuréthane ou polyuréthane-urée.

15. Segment de copolymère selon la revendication 1, dans lequel A¹ est un hydroxyle ou un groupe aminé ; A² est hydrogène, un hydroxyle ou un groupe amine, et dans lequel, lorsque t = 0 et n = 1, Q et Y² sont identiques.

16. Segment de copolymère selon la revendication 1, dans lequel A¹ est un hydroxyle ou un groupe aminé ; A² est hydrogène, un hydroxyle ou un groupe aminé ; et q est un entier de 3 à 50, dans lequel, lorsque t = 0 et n = 1, Q et Y² sont identiques.
